# EUROPEAN PATENT SPECIFICATION

(11) **EP 1 496 899 B1**
(45) Date of publication and mention of the grant of the patent: **23.06.2021**
(21) Application number: 03714273.4
(22) Date of filing: 18.03.2003
(51) Int. Cl.: A61K 9/08, A61K 47/02, A61K 47/08, A61K 31/44, A61K 33/30, A61P 31/02, A61P 31/10

(54) **USE OF MATERIALS HAVING ZINC IONOPHORIC BEHAVIOR**
VERWENDUNG VON SUBSTANZEN MIT ZINK-IONOPHORISCHEM VERHALTEN
UTILISATION DE MATIERES PRESENTANT UN COMPORTEMENT IONOPHORIQUE PAR RAPPORT AU ZINC

(30) Priority: 22.04.2002 US 374347 P
(43) Date of publication of application: 19.01.2005
(73) Proprietor: THE PROCTER & GAMBLE COMPANY, Cincinnati, OH 45202 (US); Arch Chemicals, Inc., Cheshire, CT 06410 (US)
(72) Inventor: SCHWARTZ, James, Robert, West Chester, OH 45069 (US); POISON, George, Harwinton, CT 06791 (US); TURLEY, Patricia, A., Orange, CT 06477 (US); NELSON, John, D., Bethlehem, CT 06751 (US); GAVIN, David, F., Cheshire, CT 06410 (US); ROBERTS, Katherine, P., Derby, CT 06418 (US); MARGRAF, Carl, Hinz, III, Cincinnati, OH 45226 (US); KAUFMAN, David, Joseph, Fairfield, OH 45014 (US); MARSH, Randall, Glenn, West Chester, OH 45069 (US)
(74) Representative: P&G Patent Germany
(86) International application number: PCT/US2003/008476
(87) International publication number: WO 2003/088965

(56) References cited:
- WO-A-01/00021
- WO-A-01/00151
- GB-A- 2 141 929
- US-A- 3 236 733
- US-A- 5 518 774
- US-A- 5 696 169
- SAXTON C A ET AL: "ANTIPLAQUE EFFECTS AND MODE OF ACTION OF A COMBINATION OF ZINC CITRATE AND A NONIONIC ANTIMICROBIAL AGENT" SCANDINAVIAN JOURNAL OF DENTAL RESEARCH, COPENHAGEN, DK, vol. 96, no. 3, June 1988 (1988-06), pages 212-217, XP001079620 ISSN: 0029-845X
- None

## Description

### Field

The present invention relates to methods for delivering excess zinc to eukaryotic cells to inhibit the cell metabolism. The present invention also relates to methods of treating microbial infections on the skin or scalp. The present invention further relates to methods for the treatment of dandruff, and compositions which provide improved anti-dandruff activity.

### Background

Various anti-dandruff compositions are commercially available or otherwise known in the shampoo art. These compositions typically comprise detersive surfactants and particulate, crystalline anti-microbial agents dispersed and suspended throughout the composition. Anti-microbial agents used for this purpose include sulfur, selenium sulfide and polyvalent metal salts of pyridinethione. During the shampooing process, these anti-microbial agents deposit on the scalp to provide anti-dandruff activity. Soluble anti-dandruff agents, such as ketoconazole, octopirox, and climbasole are also known in the art.

Polyvalent metal salts of pyrithione (also known as 1-hydroxy-2-pyridinethione; 2-pyridinethiol-1-oxide; 2-pyridinethione; 2-mercaptopyridine-N-oxide; pyridinethione; and pyridinethione-N-oxide) are known to be effective biocidal agents and are widely used as fungicides and bacteriocides in paints and metalworking fluids. Polyvalent metal salts of pyrithione are also used as fungicides and bacteriocides in personal care compositions such as foot powders and anti-dandruff shampoos. The polyvalent metal salts of pyrithione are only sparingly soluble in water and include magnesium pyrithione, barium pyrithione, bismuth pyrithione, strontium pyrithione, zinc pyrithione, cadmium pyrithione, and zirconium pyrithione.

Zinc pyrithione is especially useful as anti-microbial agents in personal care compositions. Zinc pyrithione is known as an anti-dandruff component in shampoos. Synthesis of polyvalent pyrithione salts is described in U.S. Patent No. 2,809,971 to Berstein, et al. Other patents disclosing similar compounds and processes for making them include U.S. Patents Nos. 2,786,847; 3,589,999; 3,590,035; and 3,773,770. WO 01/00021 A1 is related to an antimicrobial composition, comprising pyrithione or a pyrithione complex; and a zinc or copper or silver source selected from the group consisting of zinc or copper or silver salts, oxides, hydroxides, sulfates, chlorides, metals, and combinations thereof; wherein the weight ratio of the zinc or copper or silver source to the pyrithione or the pyrithione complex is in the range from 1:300 to 50:1, and wherein the antimicrobial composition has an enhanced biocidal effect against a variety of free-living microorganisms or biofilms. US 5 696 169 A refers to a method of controlling bacteria or fungi which comprises contacting therewith a zinc compound and at least one member selected from among hinokitiol and salts thereof. WO 01/00151 A sets out topical compositions for the treatment of microbial infections on the skin or scalp which include a polyvalent metal salt of pyrithione and include a metal ion source.

While pyrithione biocides have proven useful for a wide range of applications, the utility of these compounds is limited to the control of select species and strains of fungi and bacteria. Further, while higher concentrations of pyrithione salts have been observed to control the growth of a wider range of organisms, the useful amount of polyvalent metal salts of pyrithione that can be added to a commercial product is limited by efficacy and economic considerations, and environmental concerns.

Despite the options available, consumers still desire a shampoo which provides superior anti-dandruff efficacy versus currently marketed products. Such a superior efficacy can be difficult to achieve.

For example, it was previously believed that anti-dandruff efficacy could be achieved by "solubilizing" a zinc pyrithione complex in a strong chelating agent. One such approach, disclosed in European Patent Application No. 077,630 to Dixon was to "solubilize" zinc pyrithione in a strong chelating agent in the presence of divalent copper cations. However, the "solubilization" process disclosed in the '630 Application actually results in the break down of the chemical structure of the zinc pyrithione complex. The resulting composition contains a complex of the chelating agent/zinc in solution with free pyrithione ions. The free pyrithione ions are soluble in the composition. The '630 Application discloses that this approach results in a clear product that is physically stable and provides anti-dandruff benefits. Such a composition would fall outside of the current Federal Drug Administration monograph for zinc pyrithione.

### Summary

The present invention relates to a cosmetic method for delivering excess zinc to eukaryotic cells to inhibit the metabolism of the cell, the method comprising treating the cells with a composition comprising a zinc ionophoric material, wherein the zinc ionophoric material is capable of delivering a zinc ion across a cellular membrane, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof, wherein an increase in a zinc ionophoric material's antifungal activity is achieved with at least a 50% reduction in an amount of zinc ionophoric material necessary to inhibit cell growth when in the presence of 5 ppm or less of a zinc containing material and further wherein there is an increase in an intracellular zinc level by 1.5 fold more than would occur in the absence of the zinc ionophoric material.

A composition is provided and comprises a zinc ionophoric material, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof, for use in the treatment of microbial infections.

A composition is provided and comprises a zinc ionophoric material, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof, for use in the treatment of fungal infections.

A composition is provided and comprises a zinc ionophoric material, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof, for use in the treatment of dandruff.

These and other features, aspects, and advantages of the present invention will become evident to those skilled in the art from a reading of the present disclosure.

### Detailed Description

While the specification concludes with claims which particularly point out and distinctly claim the invention, it is believed the present invention will be better understood from the following description.

It has now surprisingly been found, in accordance with the present invention, that anti-dandruff efficacy can be dramatically increased in topical compositions by the use of materials exhibiting zinc ionophoric behavior. Investigations into the anti-fungal mechanism of zinc pyrithione functions have led to the hypothesis that the Zn²⁺ ion plays a very strong role in the toxicity of zinc pyrithione and pyrithione functions as a delivery vehicle for transporting Zn²⁺ to the fungal cell. This understanding has led to the conclusion that, in general, materials that facilitate the transport of Zn²⁺ to cells will be effective anti-fungals and relevant anti-dandruff technologies; these types of materials are termed materials having zinc ionophoric behavior.

An embodiment of the present invention provides a method for delivering excess zinc to eukaryotic cells to inhibit the cells metabolism by the utilization of materials having zinc ionophoric behavior. Another embodiment of the present invention relates to topical skin and/or hair compositions which provide superior anti-dandruff efficacy. These, and other embodiments, will become readily apparent from the detailed description below.

The present invention can comprise, consist of, or consist essentially of the essential elements and limitations of the invention described herein, as well any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

The components and/or steps, including those which may optionally be added, of the various embodiments of the present invention, are described in detail below.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more"
Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.

### A. Zinc Containing Material

The composition of the present invention includes an effective amount of a zinc containing material. Herein "zinc containing material" or ZCM means a material comprising zinc bound covalently, ionically, or physically by a host material.

Preferred embodiments of the present invention include from 0.001% to 10% of a zinc containing material; more preferably from 0.01% to 5%; more preferably still from 0.1% to 3%.

Examples of zinc containing include the following:
Layered structures are those with crystal growth primarily occurring in two dimensions. It is conventional to describe layer structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLM's) may have zinc incorporated in the layers and/or as more labile components of the gallery ions.

Many ZLM's occur naturally as minerals. Common examples include hydrozincite (zinc carbonate hydroxide), basic zinc carbonate, aurichalcite (zinc copper carbonate hydroxide), rosasite (copper zinc carbonate hydroxide) and many related minerals that are zinc-containing. Natural ZLM's can also occur wherein anionic layer species such as clay-type minerals (e.g., phyllosilicates) contain ion-exchanged zinc gallery ions. All of these natural materials can also be obtained synthetically or formed in situ in a composition or during a production process.

Another common class of ZLM's, which are often, but not always, synthetic, is layered doubly hydroxides, which are generally represented by the formula [M²⁺₁₋ₓM³⁺ₓ(OH)₂]^{x+} A^{m-}_{x/m}·nH₂O and some or all of the divalent ions (M²⁺) would be represented as zinc ions (Crepaldi, EL, Pava, PC, Tronto, J, Valim, JB J. Colloid Interfac. Sci. 2002, 248, 429-42).

Yet another class of ZLM's can be prepared called hydroxy double salts (Morioka, H., Tagaya, H., Karasu, M, Kadokawa, J, Chiba, K Inorg. Chem. 1999, 38, 4211-6). Hydroxy double salts can be represented by the general formula [M²⁺₁₋ₓM²⁺₁₊ₓ(OH)_{3(1-y})]⁺ Aⁿ⁻_{(1=3y)/n}·nH₂O where the two metal ion may be different; if they are the same and represented by zinc, the formula simplifies to [Zn₁₊ₓ(OH)₂]^{2x+} 2x A⁻·nH₂O. This latter formula represents (where x=0.4) common materials such as zinc hydroxychloride and zinc hydroxynitrate. These are related to hydrozincite as well wherein a divalent anion replace the monovalent anion. These materials can also be formed in situ in a composition or in or during a production process.

Commercially available sources of zinc carbonate include Zinc Carbonate Basic (Cater Chemicals: Bensenville, IL, USA), Zinc Carbonate (Shepherd Chemicals: Norwood, OH, USA), Zinc Carbonate (CPS Union Corp.: New York, NY, USA), Zinc Carbonate (Elementis Pigments: Durham, UK), and Zinc Carbonate AC (Bruggemann Chemical: Newtown Square, PA, USA).

### B. Zinc Ionophoric Material (ZIM)

The composition further includes a zinc ionophoric material. Herein, "zinc ionophoric material" and "ZIM" means a material which is a hydrophobic molecule capable of increasing cell permeability to zinc ions (i.e., exhibiting zinc ionophoric behavior). Without being bound by theory, it is believed that ZIMs shield the charge of the zinc ion to be transported, enabling it to penetrate the hydrophobic interior of the lipid bilayer. ZIMs may be channel-forming ionophores or mobile ion carriers. ZIMs may be those commonly known as zinc ionophores or those that are hydrophobic zinc chelators that possess zinc ionophoric behavior. Hydrophobic zinc chelators are materials that bind zinc and increase hydrophobicity of zinc ions such that, for example, it will partition into non-aqueous solvents. ZIMs can be effective including zinc being present in the composition or zinc being available within the system wherein a ZIM is present, yet preferred ZIMs contain zinc ions; i.e, zinc salt forms of materials exhibiting zinc ionophoric behavior.

Preferred embodiments include from 0.01% to 5% of a ZIM; more preferably from 0.1% to 2%.

In embodiments having a zinc containing material and a ZIM, the ratio of zinc containing material to ZIM is preferably from 5:100 to 5:1; more preferably from 2:10 to 3:1; more preferably still from 1:2 to 2:1.

In preferred embodiments of the present invention, the ZIM has a potency against target microorganisms such that the minimum inhibitory concentration ("MIC") is below 5000 parts per million. The MIC is a measurement well understood by those skilled in the art and is indicative of anti-fungal efficacy. Generally, the lower the value of the composition, the better its anti-fungal efficacy, due to increased inherent ability of the anti-dandruff agent to inhibit the growth of microorganisms. The lowest tested dilution of anti-microbial active that yields no growth is defined as the MIC.

The ZIM is the zinc salt of 1-hydroxy-2-pyridinethione (known as "zinc pyrithione" or "ZPT"); more preferably ZPT in platelet particle form, wherein the particles have an average size of up to 20µm, preferably up to 5µm, more preferably up to 2.5µm.

Pyridinethione anti-microbial and anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

It is further contemplated that when ZPT is used as the anti-microbial particulate in the anti-microbial compositions herein, that an additional benefit of hair growth or re-growth may be stimulated or regulated, or both, or that hair loss may be reduced or inhibited, or that hair may appear thicker or fuller.

Zinc pyrithione may be made by reacting 1-hydroxy-2-pyridinethione (i.e., pyrithione acid) or a soluble salt thereof with a zinc salt (e.g. zinc sulfate) to form a zinc pyrithione precipitate, as illustrated in U.S. Patent No. 2,809,971.

### C. Topical Carrier

In a preferred embodiment, the composition of the present invention is in the form of a topical compositions, which includes a topical carrier. Preferably, the topical carrier is selected from a broad range of traditional personal care carriers depending on the type of composition to be formed. By suitable selections of compatible carriers, it is contemplated that such a composition is prepared in the form of daily skin or hair products including skin lotions or hair rinses, daily hair-grooming products, such as hair lotions, hair sprays, hair tonics, conditioning treatments, gels, mousses and dressings, and the like, or they may be prepared in the form of cleansing products, such as hair and/or scalp shampoos, body washes, hand cleansers, waterless hand sanitizer/cleansers, and the like.

Preferably, the topical carrier is water, a common organic solvent, or mixtures thereof. Suitable common organic solvents are C₂-C₃ lower monohydric or polyhydric alcohols such as ethanol, propanol, isopropanol, glycerine, dimethylformamide, dimethylacetamide, and dimethylsulfoxide.

In a preferred embodiment, the carrier is water. Preferably the compositions of the present invention comprise from 40% to 95% water by weight of the composition; preferably from 50% to 85%, more preferably still from 60% to 80%.

In another embodiment of the present invention, the composition is in the form of a solid powder for application to the skin. Such a powder may comprise a solid cosmetic carrier. The solid cosmetic carrier may be talc, which is a hydrated magnesium silicate, used in the form of particles generally less than 40 µm in size; micas, which are aluminosilicates compositions, which exist in the form of scales which are 2 to 200 µm; modified or unmodified starch, in particular rice starch; silica; alumina; boron nitride;
kaolin, which is a hydrated aluminum silicate; zinc and titanium oxides; precipitated calcium carbonate; magnesium carbonate or hydrocarbonate; metallic soaps derived from a carboxylic organic acid having 8 to 22 carbon atoms, for example zinc, magnesium or lithium stearate, zinc laurate, magnesium myristate and the like; synthetic polymer (or copolymer) powders chosen from polyethylene and its derivatives, for example polytetrafluoroethylene, polystyrene and the like; polyacrylates, polymethacrylates, polyesters or polyamides and the like, for example nylon powders; and powders in the form of hollow microspheres made from thermoplastic synthetic material, whose hollow part contains a gas.

All percentages are by weight of total composition unless specifically stated otherwise.

### D. Detersive Surfactant

The composition of the present invention includes a detersive surfactant. The detersive surfactant component is included to provide cleaning performance to the composition. The detersive surfactant component in turn comprises anionic detersive surfactant, zwitterionic or amphoteric detersive surfactant, or a combination thereof. Such surfactants should be physically and chemically compatible with the essential components described herein, or should not otherwise unduly impair product stability, aesthetics or performance.

Suitable anionic detersive surfactant components for use in the composition herein include those which are known for use in hair care or other personal care cleansing compositions. The concentration of the anionic surfactant component in the composition should be sufficient to provide the desired cleaning and lather performance, and generally range from 5% to 50%, preferably from 8% to 30%, more preferably from 10% to 25%, even more preferably from 12% to 22%.

Preferred anionic surfactants suitable for use in the compositions are the alkyl and alkyl ether sulfates. These materials have the respective formulae ROSO₃M and RO(C₂H₄O)ₓSO₃M, wherein R is alkyl or alkenyl of from 8 to 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium.

Preferably, R has from 8 to 18 carbon atoms, more preferably from 10 to 16 carbon atoms, even more preferably from 12 to 14 carbon atoms, in both the alkyl and alkyl ether sulfates. The alkyl ether sulfates are typically made as condensation products of ethylene oxide and monohydric alcohols having from 8 to 24 carbon atoms. The alcohols can be synthetic or they can be derived from fats, e.g., coconut oil, palm kernel oil, tallow. Lauryl alcohol and straight chain alcohols derived from coconut oil or palm kernel oil are preferred. Such alcohols are reacted with between 0 and 10, preferably from 2 to 5, more preferably 3, molar proportions of ethylene oxide, and the resulting mixture of molecular species having, for example, an average of 3 moles of ethylene oxide per mole of alcohol, is sulfated and neutralized.

Other suitable anionic detersive surfactants are the water-soluble salts of organic, sulfuric acid reaction products conforming to the formula [R¹-SO₃-M] where R¹ is a straight or branched chain, saturated, aliphatic hydrocarbon radical having from 8 to 24, preferably 10 to 18, carbon atoms; and M is a cation described hereinbefore.

Still other suitable anionic detersive surfactants are the reaction products of fatty acids esterified with isethionic acid and neutralized with sodium hydroxide where, for example, the fatty acids are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Pat. Nos. 2,486,921; 2,486,922; and 2,396,278.

Other anionic detersive surfactants suitable for use in the compositions are the succinnates, examples of which include disodium N-octadecylsulfosuccinnate; disodium lauryl sulfosuccinate; diammonium lauryl sulfosuccinate; tetrasodium N-(1,2-dicarboxyethyl)-N-octadecylsulfosuccinnate; diamyl ester of sodium sulfosuccinic acid; dihexyl ester of sodium sulfosuccinic acid; and dioctyl esters of sodium sulfosuccinic acid.

Other suitable anionic detersive surfactants include olefin sulfonates having 10 to 24 carbon atoms. In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process. A non limiting example of such an alpha-olefin sulfonate mixture is described in U.S. Patent 3,332,880.

Another class of anionic detersive surfactants suitable for use in the compositions are the beta-alkyloxy alkane sulfonates. These surfactants conform to the formula 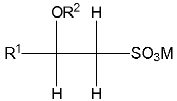 where R¹ is a straight chain alkyl group having from 6 to 20 carbon atoms, R² is a lower alkyl group having from 1 to 3 carbon atoms, preferably 1 carbon atom, and M is a water-soluble cation as described hereinbefore.

Preferred anionic detersive surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof.

Suitable amphoteric or zwitterionic detersive surfactants for use in the composition herein include those which are known for use in hair care or other personal care cleansing. Concentration of such amphoteric detersive surfactants preferably ranges from 0.5% to 20%, preferably from 1 % to 10%. Non limiting examples of suitable zwitterionic or amphoteric surfactants are described in U.S. Pat. Nos. 5,104,646 (Bolich Jr. et al.), 5,106,609 (Bolich Jr. et al.).

Amphoteric detersive surfactants suitable for use in the composition are well known in the art, and include those surfactants broadly described as derivatives of aliphatic secondary and tertiary amines in which the aliphatic radical can be straight or branched chain and wherein one of the aliphatic substituents contains from 8 to 18 carbon atoms and one contains an anionic group such as carboxy, sulfonate, sulfate, phosphate, or phosphonate. Preferred amphoteric detersive surfactants for use in the present invention include cocoamphoacetate, cocoamphodiacetate, lauroamphoacetate, lauroamphodiacetate, and mixtures thereof.

Zwitterionic detersive surfactants suitable for use in the composition are well known in the art, and include those surfactants broadly described as derivatives of aliphatic quaternary ammonium, phosphonium, and sulfonium compounds, in which the aliphatic radicals can be straight or branched chain, and wherein one of the aliphatic substituents contains from 8 to 18 carbon atoms and one contains an anionic group such as carboxy, sulfonate, sulfate, phosphate or phosphonate. Zwitterionics such as betaines are preferred.

The compositions of the present invention may further comprise additional surfactants for use in combination with the anionic detersive surfactant component described hereinbefore. Suitable optional surfactants include nonionic and cationic surfactants. Any such surfactant known in the art for use in hair or personal care products may be used, provided that the optional additional surfactant is also chemically and physically compatible with the essential components of the composition, or does not otherwise unduly impair product performance, aesthetics or stability. The concentration of the optional additional surfactants in the composition may vary with the cleansing or lather performance desired, the optional surfactant selected, the desired product concentration, the presence of other components in the composition, and other factors well known in the art.

Non limiting examples of other anionic, zwitterionic, amphoteric or optional additional surfactants suitable for use in the compositions are described in McCutcheon's, Emulsifiers and Detergents, 1989 Annual, published by M. C. Publishing Co., and U.S. Pat. Nos. 3,929,678, 2,658,072; 2,438,091; 2,528,378.

### E. Dispersed Particles

The composition of the present invention may include dispersed particles. In the compositions of the present invention, it is preferable to incorporate at least 0.025% by weight of the dispersed particles, more preferably at least 0.05%, still more preferably at least 0.1%, even more preferably at least 0.25%, and yet more preferably at least 0.5% by weight of the dispersed particles. In the compositions of the present invention, it is preferable to incorporate no more than 20% by weight of the dispersed particles, more preferably no more than 10%, still more preferably no more than 5%, even more preferably no more than 3%, and yet more preferably no more than 2% by weight of the dispersed particles.

### F. Aqueous Carrier

The compositions of the present invention are typically in the form of pourable liquids (under ambient conditions). The compositions will therefore typically comprise an aqueous carrier, which is present at a level of from 20% to 95%, preferably from 60% to 85%. The aqueous carrier may comprise water, or a miscible mixture of water and organic solvent, but preferably comprises water with minimal or no significant concentrations of organic solvent, except as otherwise incidentally incorporated into the composition as minor ingredients of other essential or optional components.

### G. Additional Components

The compositions of the present invention may further comprise one or more optional components known for use in hair care or personal care products, provided that the optional components are physically and chemically compatible with the essential components described herein, or do not otherwise unduly impair product stability, aesthetics or performance. Individual concentrations of such optional components may range from 0.001% to 10%.

Non-limiting examples of optional components for use in the composition include cationic polymers, conditioning agents (hydrocarbon oils, fatty esters, silicones), anti dandruff agents, suspending agents, viscosity modifiers, dyes, nonvolatile solvents or diluents (water soluble and insoluble), pearlescent aids, foam boosters, additional surfactants or nonionic cosurfactants, pediculocides, pH adjusting agents, perfumes, preservatives, chelants, proteins, skin active agents, sunscreens, UV absorbers, vitamins, minerals, herbal/fruit/food extracts, sphingolipids derivatives or synthetical derivative, and clay.

### 1. Cationic Polymers

The compositions of the present invention may contain a cationic polymer. Concentrations of the cationic polymer in the composition typically range from 0.05% to 3%, preferably from 0.075% to 2.0%, more preferably from 0.1% to 1.0%. Preferred cationic polymers will have cationic charge densities of at least 0.9 meq/gm, preferably at least 1.2 meq/gm, more preferably at least 1.5 meq/gm, but also preferably less than 7 meq/gm, more preferably less than 5 meq/gm, at the pH of intended use of the composition, which pH will generally range from pH 3 to pH 9, preferably between pH 4 and pH 8. Herein, "cationic charge density" of a polymer refers to the ratio of the number of positive charges on the polymer to the molecular weight of the polymer. The average molecular weight of such suitable cationic polymers will generally be between 10,000 and 10 million, preferably between 50,000 and 5 million, more preferably between 100,000 and 3 million.

Suitable cationic polymers for use in the compositions of the present invention contain cationic nitrogen-containing moieties such as quaternary ammonium or cationic protonated amino moieties. The cationic protonated amines can be primary, secondary, or tertiary amines (preferably secondary or tertiary), depending upon the particular species and the selected pH of the composition. Any anionic counterions can be used in association with the cationic polymers so long as the polymers remain soluble in water, in the composition, or in a coacervate phase of the composition, and so long as the counterions are physically and chemically compatible with the essential components of the composition or do not otherwise unduly impair product performance, stability or aesthetics. Non limiting examples of such counterions include halides (e.g., chloride, fluoride, bromide, iodide), sulfate and methylsulfate.

Non limiting examples of such polymers are described in the CTFA Cosmetic Ingredient Dictionary, 3rd edition, edited by Estrin, Crosley, and Haynes, (The Cosmetic, Toiletry, and Fragrance Association, Inc., Washington, D.C. (1982)).

Non limiting examples of suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

Suitable cationic protonated amino and quaternary ammonium monomers, for inclusion in the cationic polymers of the composition herein, include vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts.

Other suitable cationic polymers for use in the compositions include copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16); copolymers of 1-vinyl-2-pyrrolidone and dimethylaminoethyl methacrylate (referred to in the industry by CTFA as Polyquaternium-11); cationic diallyl quaternary ammonium-containing polymers, including, for example, dimethyldiallylammonium chloride homopolymer, copolymers of acrylamide and dimethyldiallylammonium chloride (referred to in the industry by CTFA as Polyquaternium 6 and Polyquaternium 7, respectively); amphoteric copolymers of acrylic acid including copolymers of acrylic acid and dimethyldiallylammonium chloride (referred to in the industry by CTFA as Polyquaternium 22), terpolymers of acrylic acid with dimethyldiallylammonium chloride and acrylamide (referred to in the industry by CTFA as Polyquaternium 39), and terpolymers of acrylic acid with methacrylamidopropyl trimethylammonium chloride and methylacrylate (referred to in the industry by CTFA as Polyquaternium 47). Preferred cationic substituted monomers are the cationic substituted dialkylaminoalkyl acrylamides, dialkylaminoalkyl methacrylamides, and combinations thereof. These preferred monomers conform the to the formula 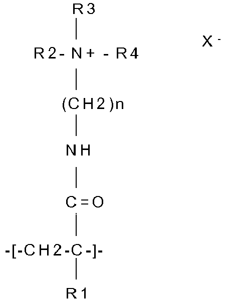 wherein R¹ is hydrogen, methyl or ethyl; each of R², R³ and R⁴ are independently hydrogen or a short chain alkyl having from 1 to 8 carbon atoms, preferably from 1 to 5 carbon atoms, more preferably from 1 to 2 carbon atoms; n is an integer having a value of from 1 to 8, preferably from 1 to 4; and X is a counterion. The nitrogen attached to R², R³ and R⁴ may be a protonated amine (primary, secondary or tertiary), but is preferably a quaternary ammonium wherein each of R², R³ and R⁴ are alkyl groups a non limiting example of which is polymethyacrylamidopropyl trimonium chloride, available under the trade name Polycare 133, from Rhone-Poulenc, Cranberry, N.J., U.S.A.

Other suitable cationic polymers for use in the composition include polysaccharide polymers, such as cationic cellulose derivatives and cationic starch derivatives. Suitable cationic polysaccharide polymers include those which conform to the formula 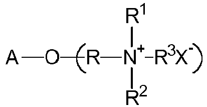 wherein A is an anhydroglucose residual group, such as a starch or cellulose anhydroglucose residual; R is an alkylene oxyalkylene, polyoxyalkylene, or hydroxyalkylene group, or combination thereof; R1, R2, and R3 independently are alkyl, aryl, alkylaryl, arylalkyl, alkoxyalkyl, or alkoxyaryl groups, each group containing up to 18 carbon atoms, and the total number of carbon atoms for each cationic moiety (i.e., the sum of carbon atoms in R1, R2 and R3) preferably being 20 or less; and X is an anionic counterion as described in hereinbefore.

Preferred cationic cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA) in their Polymer LR, JR, and KG series of polymers. Other suitable types of cationic cellulose includes the polymeric quaternary ammonium salts of hydroxyethyl cellulose reacted with lauryl dimethyl ammonium-substituted epoxide referred to in the industry (CTFA) as Polyquaternium 24. These materials are available from Amerchol Corp. under the tradename Polymer LM-200.

Other suitable cationic polymers include cationic guar gum derivatives, such as guar hydroxypropyltrimonium chloride, specific examples of which include the Jaguar series commercially avaialable from Rhone-Poulenc Incorporated and the N-Hance series commercially available from Aqualon Division of Hercules, Inc. Other suitable cationic polymers include quaternary nitrogen-containing cellulose ethers, some examples of which are described in U.S. Pat. No. 3,962,418. Other suitable cationic polymers include copolymers of etherified cellulose, guar and starch, some examples of which are described in U.S. Pat. No. 3,958,581. When used, the cationic polymers herein are either soluble in the composition or are soluble in a complex coacervate phase in the composition formed by the cationic polymer and the anionic, amphoteric and/or zwitterionic detersive surfactant component described hereinbefore. Complex coacervates of the cationic polymer can also be formed with other charged materials in the composition.

Techniques for analysis of formation of complex coacervates are known in the art. For example, microscopic analyses of the compositions, at any chosen stage of dilution, can be utilized to identify whether a coacervate phase has formed. Such coacervate phase will be identifiable as an additional emulsified phase in the composition. The use of dyes can aid in distinguishing the coacervate phase from other insoluble phases dispersed in the composition.

### 2. Nonionic polymers

Polyalkylene glycols having a molecular weight of more than 1000 are useful herein. Useful are those having the following general formula: 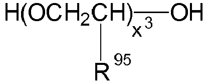 wherein R⁹⁵ is selected from the group consisting of H, methyl, and mixtures thereof. Polyethylene glycol polymers useful herein are PEG-2M (also known as Polyox WSR® N-10, which is available from Union Carbide and as PEG-2,000); PEG-5M (also known as Polyox WSR® N-35 and Polyox WSR® N-80, available from Union Carbide and as PEG-5,000 and Polyethylene Glycol 300,000); PEG-7M (also known as Polyox WSR® N-750 available from Union Carbide); PEG-9M (also known as Polyox WSR® N-3333 available from Union Carbide); and PEG-14 M (also known as Polyox WSR® N-3000 available from Union Carbide).

### 3. Conditioning agents

Conditioning agents include any material which is used to give a particular conditioning benefit to hair and/or skin. In hair treatment compositions, suitable conditioning agents are those which deliver one or more benefits relating to shine, softness, combability, antistatic properties, wet-handling, damage, manageability, body, and greasiness. The conditioning agents useful in the compositions of the present invention typically comprise a water insoluble, water dispersible, non-volatile, liquid that forms emulsified, liquid particles. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein. Such conditioning agents should be physically and chemically compatible with the essential components of the composition, and should not otherwise unduly impair product stability, aesthetics or performance.

The concentration of the conditioning agent in the composition should be sufficient to provide the desired conditioning benefits, and as will be apparent to one of ordinary skill in the art. Such concentration can vary with the conditioning agent, the conditioning performance desired, the average size of the conditioning agent particles, the type and concentration of other components, and other like factors.

### 1. Silicones

The conditioning agent of the compositions of the present invention is preferably an insoluble silicone conditioning agent. The silicone conditioning agent particles may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile silicone materials ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

The concentration of the silicone conditioning agent typically ranges from 0.01% to 10%, preferably from 0.1% to 8%, more preferably from 0.1% to 5%, more preferably from 0.2% to 3%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions of the present invention preferably have a viscosity, as measured at 25°C, from 20 to 2,000,000 centistokes ("csk"), more preferably from 1,000 to 1,800,000 csk, even more preferably from 50,000 to 1,500,000 csk, more preferably from 100,000 to 1,500,000 csk.

The dispersed silicone conditioning agent particles typically have a number average particle diameter ranging from 0.01µm to 50µm. For small particle application to hair, the number average particle diameters typically range from 0.01 µm to 4µm, preferably from 0.01µm to 2µm, more preferably from 0. 01µm to 0.5µm. For larger particle application to hair, the number average particle diameters typically range from 4µm to 50µm, preferably from 6µm to 30µm, more preferably from 9µm to 20µm, more preferably from 12µm to 18µm.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

### a. Silicone oils

Silicone fluids include silicone oils, which are flowable silicone materials having a viscosity, as measured at 25°C, less than 1,000,000 csk, preferably from 5 csk to 1,000,000 csk, more preferably from 100 csk to 600,000 csk. Suitable silicone oils for use in the compositions of the present invention include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used.

Silicone oils include polyalkyl or polyaryl siloxanes which conform to the following Formula (III): 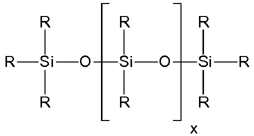 wherein R is aliphatic, preferably alkyl or alkenyl, or aryl, R can be substituted or unsubstituted, and x is an integer from 1 to 8,000. Suitable R groups for use in the compositions of the present invention include, but are not limited to: alkoxy, aryloxy, alkaryl, arylalkyl, arylalkenyl, alkamino, and ether-substituted, hydroxyl-substituted, and halogen-substituted aliphatic and aryl groups. Suitable R groups also include cationic amines and quaternary ammonium groups.

Preferred alkyl and alkenyl substituents are C₁ to C₅ alkyls and alkenyls, more preferably from C₁ to C₄, more preferably from C₁ to C₂. The aliphatic portions of other alkyl-, alkenyl-, or alkynyl-containing groups (such as alkoxy, alkaryl, and alkamino) can be straight or branched chains, and are preferably from C₁ to C₅, more preferably from C₁ to C₄, even more preferably from C₁ to C₃, more preferably from C₁ to C₂. As discussed above, the R substituents can also contain amino functionalities (e.g. alkamino groups), which can be primary, secondary or tertiary amines or quaternary ammonium. These include mono-, di- and tri- alkylamino and alkoxyamino groups, wherein the aliphatic portion chain length is preferably as described herein.

### b. Amino and Cationic silicones

Cationic silicone fluids suitable for use in the compositions of the present invention include, but are not limited to, those which conform to the general formula (V):

(R₁)ₐG₃₋ₐ-Si-(-OSiG₂)ₙ-(-OSiG_{b}(R₁)_{2-b)m}-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 0; b is 0 or 1, preferably 1; n is a number from 0 to 1,999, preferably from 49 to 499; m is an integer from 1 to 2,000, preferably from 1 to 10; the sum of n and m is a number from 1 to 2,000, preferably from 50 to 500; R¹ is a monovalent radical conforming to the general formula CqH_{2q}L, wherein q is an integer having a value from 2 to 8 and L is selected from the following groups:

-N(R₂)CH₂-CH₂-N(R₂)₂

-N(R₂)₂

-N(R₂)₃A⁻

-N(R₂)CH₂-CH₂-NR₂H₂A⁻

wherein R² is hydrogen, phenyl, benzyl, or a saturated hydrocarbon radical, preferably an alkyl radical from C₁ to C₂₀, and A⁻ is a halide ion.

An especially preferred cationic silicone corresponding to formula (V) is the polymer known as "trimethylsilylamodimethicone", which is shown below in formula (VI):

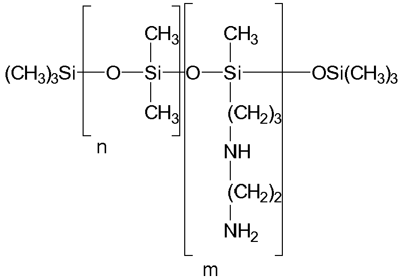

Other silicone cationic polymers which may be used in the compositions of the present invention are represented by the general formula (VII): 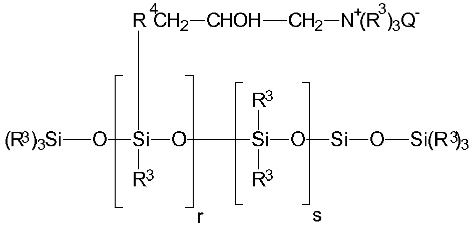 wherein R³ is a monovalent hydrocarbon radical from C₁ to C₁₈, preferably an alkyl or alkenyl radical, such as methyl; R⁴ is a hydrocarbon radical, preferably a C₁ to C₁₈ alkylene radical or a C₁₀ to C₁₈ alkyleneoxy radical, more preferably a C₁ to C₈ alkyleneoxy radical; Q⁻ is a halide ion, preferably chloride; r is an average statistical value from 2 to 20, preferably from 2 to 8; s is an average statistical value from 20 to 200, preferably from 20 to 50. A preferred polymer of this class is known as UCARE SILICONE ALE 56™, available from Union Carbide.

### c. Silicone gums

Other silicone fluids suitable for use in the compositions of the present invention are the insoluble silicone gums. These gums are polyorganosiloxane materials having a viscosity, as measured at 25°C, of greater than or equal to 1,000,000 csk. Silicone gums are described in U.S. Pat. No. 4,152,416; Noll and Walter, Chemistry and Technology of Silicones, New York: Academic Press (1968); and in General Electric Silicone Rubber Product Data Sheets SE 30, SE 33, SE 54 and SE 76. Specific non-limiting examples of silicone gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

### d. High refractive index silicones

Other non-volatile, insoluble silicone fluid conditioning agents that are suitable for use in the compositions of the present invention are those known as "high refractive index silicones," having a refractive index of at least 1.46, preferably at least 1.48, more preferably at least 1.52, more preferably at least 1.55. The refractive index of the polysiloxane fluid will generally be less than 1.70, typically less than 1.60. In this context, polysiloxane "fluid" includes oils as well as gums.

The high refractive index polysiloxane fluid includes those represented by general Formula (III) above, as well as cyclic polysiloxanes such as those represented by Formula (VIII) below: 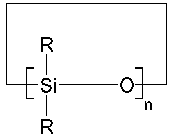 wherein R is as defined above, and n is a number from 3 to 7, preferably from 3 to 5.

The high refractive index polysiloxane fluids contain an amount of aryl-containing R substituents sufficient to increase the refractive index to the desired level, which is described herein. Additionally, R and n must be selected so that the material is non-volatile.

Aryl-containing substituents include those which contain alicyclic and heterocyclic five and six member aryl rings and those which contain fused five or six member rings. The aryl rings themselves can be substituted or unsubstituted.

Generally, the high refractive index polysiloxane fluids will have a degree of aryl-containing substituents of at least 15%, preferably at least 20%, more preferably at least 25%, even more preferably at least 35%, more preferably at least 50%. Typically, the degree of aryl substitution will be less than 90%, more generally less than 85%, preferably from 55% to 80%.

Preferred high refractive index polysiloxane fluids have a combination of phenyl or phenyl derivative substituents (more preferably phenyl), with alkyl substituents, preferably C₁-C₄ alkyl (more preferably methyl), hydroxy, or C₁-C₄ alkylamino (especially -R¹NHR²NH2 wherein each R¹ and R² independently is a C₁-C₃ alkyl, alkenyl, and/or alkoxy).

When high refractive index silicones are used in the compositions of the present invention, they are preferably used in solution with a spreading agent, such as a silicone resin or a surfactant, to reduce the surface tension by a sufficient amount to enhance spreading and thereby enhance the glossiness (subsequent to drying) of hair treated with the compositions.

Silicone fluids suitable for use in the compositions of the present invention are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984).

### e. Silicone resins

Silicone resins may be included in the silicone conditioning agent of the compositions of the present invention. These resins are highly cross-linked polymeric siloxane systems. The cross-linking is introduced through the incorporation of trifunctional and tetrafunctional silanes with monofunctional or difunctional, or both, silanes during manufacture of the silicone resin.

Silicone materials and silicone resins in particular, can conveniently be identified according to a shorthand nomenclature system known to those of ordinary skill in the art as "MDTQ" nomenclature. Under this system, the silicone is described according to presence of various siloxane monomer units which make up the silicone. Briefly, the symbol M denotes the monofunctional unit (CH₃)₃SiO_{0.5}; D denotes the difunctional unit (CH₃)₂SiO; T denotes the trifunctional unit (CH₃)SiO₁₅; and Q denotes the quadra- or tetra-functional unit SiO₂. Primes of the unit symbols (e.g. M', D', T', and Q') denote substituents other than methyl, and must be specifically defined for each occurrence.

Preferred silicone resins for use in the compositions of the present invention include, but are not limited to MQ, MT, MTQ, MDT and MDTQ resins. Methyl is a preferred silicone substituent. Especially preferred silicone resins are MQ resins, wherein the M:Q ratio is from 0.5:1.0 to 1.5:1.0 and the average molecular weight of the silicone resin is from 1000 to 10,000.

The weight ratio of the non-volatile silicone fluid, having refractive index below 1.46, to the silicone resin component, when used, is preferably from 4:1 to 400:1, more preferably from 9:1 to 200:1, more preferably from 19:1 to 100:1, particularly when the silicone fluid component is a polydimethylsiloxane fluid or a mixture of polydimethylsiloxane fluid and polydimethylsiloxane gum as described herein. Insofar as the silicone resin forms a part of the same phase in the compositions hereof as the silicone fluid, i.e. the conditioning active, the sum of the fluid and resin should be included in determining the level of silicone conditioning agent in the composition.

### 2. Organic conditioning oils

The conditioning component of the compositions of the present invention may also comprise from 0.05% to 3%, preferably from 0.08% to 1.5%, more preferably from 0.1% to 1%, of at least one organic conditioning oil as the conditioning agent, either alone or in combination with other conditioning agents, such as the silicones (described herein).

### a. Hydrocarbon oils

Suitable organic conditioning oils for use as conditioning agents in the compositions of the present invention include, but are not limited to, hydrocarbon oils having at least 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from C₁₂ to C₁₉. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation. The concentration of such hydrocarbon oils in the composition preferably range from 0.05% to 20%, more preferably from 0.08% to 1.5%, and even more preferably from 0.1% to 1%.

### b. Polyolefins

Organic conditioning oils for use in the compositions of the present invention can also include liquid polyolefins, more preferably liquid poly-α-olefins, more preferably hydrogenated liquid poly-α-olefins. Polyolefins for use herein are prepared by polymerization of C₄ to C₁₄ olefenic monomers, preferably from C₆ to C₁₂.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents. Preferred hydrogenated α-olefin monomers include, but are not limited to: 1-hexene to 1-hexadecenes, 1-octene to 1-tetradecene, and mixtures thereof.

### c. Fatty Esters

Other suitable organic conditioning oils for use as the conditioning agent in the compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.).

Specific examples of preferred fatty esters include, but are not limited to: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the compositions of the present invention are monocarboxylic acid esters of the general formula R'COOR, wherein R' and R are alkyl or alkenyl radicals, and the sum of carbon atoms in R' and R is at least 10, preferably at least 22.

Still other fatty esters suitable for use in the compositions of the present invention are di- and tri-alkyl and alkenyl esters of carboxylic acids, such as esters of C₄ to C₈ dicarboxylic acids (e.g. C₁ to C₂₂ esters, preferably C₁ to C₆, of succinic acid, glutaric acid, and adipic acid). Specific non-limiting examples of di- and tri- alkyl and alkenyl esters of carboxylic acids include isocetyl stearyol stearate, diisopropyl adipate, and tristearyl citrate.

Other fatty esters suitable for use in the compositions of the present invention are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Still other fatty esters suitable for use in the compositions of the present invention are glycerides, including, but not limited to, mono-, di-, and tri-glycerides, preferably di- and tri-glycerides, more preferably triglycerides. For use in the compositions described herein, the glycerides are preferably the mono-, di-, and tri-esters of glycerol and long chain carboxylic acids, such as C₁₀ to C₂₂ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

Other fatty esters suitable for use in the compositions of the present invention are water insoluble synthetic fatty esters. Some preferred synthetic esters conform to the general Formula (IX): 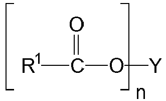 wherein R¹ is a C₇ to C₉ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from 2 to 20 carbon atoms, preferably from 3 to 14 carbon atoms. Other preferred synthetic esters conform to the general Formula (X): 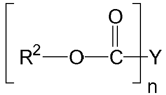 wherein R² is a C₈ to C₁₀ alkyl, alkenyl, hydroxyalkyl or hydroxyalkenyl group; preferably a saturated alkyl group, more preferably a saturated, linear, alkyl group; n and Y are as defined above in Formula (X).

Specific non-limiting examples of suitable synthetic fatty esters for use in the compositions of the present invention include: P-43 (C₈-C₁₀ triester of trimethylolpropane), MCP-684 (tetraester of 3,3 diethanol-1,5 pentadiol), MCP 121 (C₈-C₁₀ diester of adipic acid), all of which are available from Mobil Chemical Company.

### 3. Other conditioning agents

Also suitable for use in the compositions herein are the conditioning agents described by the Procter & Gamble Company in U.S. Pat. Nos. 5,674,478, and 5,750,122. Also suitable for use herein are those conditioning agents described in U.S. Pat. Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

### 4. Additional Components

The compositions of the present invention may further include a variety of additional useful components. Preferred additional components include those discussed below:

### 1. Other Anti-Microbial Actives

The compositions of the present invention may further include one or more anti-fungal or anti-microbial actives in addition to the metal pyrithione salt actives. Suitable anti-microbial actives include coal tar, sulfur, whitfield's ointment, castellani's paint, aluminum chloride, gentian violet, octopirox (piroctone olamine), ciclopirox olamine, undecylenic acid and it's metal salts, potassium permanganate, selenium sulfide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Preferred anti-microbials include itraconazole, ketoconazole, selenium sulphide and coal tar.

### a. Azoles

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from 0.01% to 5%, preferably from 0.1% to 3%, and more preferably from 0.3% to 2%, by weight of the composition. Especially preferred herein is ketoconazole.

### b. Selenium Sulfide

Selenium sulfide is a particulate anti-dandruff agent suitable for use in the anti-microbial compositions of the present invention, effective concentrations of which range from 0.1% to 4%, by weight of the composition, preferably from 0.3% to 2.5%, more preferably from 0.5% to 1.5%. Selenium sulfide is generally regarded as a compound having one mole of selenium and two moles of sulfur, although it may also be a cyclic structure that conforms to the general formula SeₓS_{y}, wherein x + y = 8. Average particle diameters for the selenium sulfide are typically less than 15µm, as measured by forward laser light scattering device (e.g. Malvern 3600 instrument), preferably less than 10 µm. Selenium sulfide compounds are described, for example, in U.S. Pat. No. 2,694,668; U.S. Pat. No. 3,152,046; U.S. Pat. No. 4,089,945; and U.S. Pat. No. 4,885,107.

### c. Sulfur

Sulfur may also be used as a particulate anti-microbial/anti-dandruff agent in the anti-microbial compositions of the present invention. Effective concentrations of the particulate sulfur are typically from 1% to 4%, by weight of the composition, preferably from 2% to 4%.

### d. Keratolytic Agents

The present invention may further comprise one or more keratolytic agents such as Salicylic Acid.

Additional anti-microbial actives of the present invention may include extracts of melaleuca (tea tree) and charcoal. The present invention may also comprise combinations of anti-microbial actives. Such combinations may include octopirox and zinc pyrithione combinations, pine tar and sulfur combinations, salicylic acid and zinc pyrithione combinations, octopirox and climbasole combinations, and salicylic acid and octopirox combinations, and mixtures thereof.

### 2. Hair loss prevention and Hair Growth Agents

The present invention may further comprise materials useful for hair loss prevention and hair growth stimulants or agents. Examples of such agents are Anti-Androgens such as Propecia, Dutasteride, RU5884; Anti-Inflammatories such as Glucocortisoids, Macrolides, Macrolides; Anti-Microbials such as Zinc pyrithione, Ketoconazole, Selenium sulfide, Acne Treatments; Immunosuppressives such as FK-506, Cyclosporin; Vasodilators such as minoxidil, Aminexil® and combinations thereof.

### 3. Sensates

The present invention may further comprise topical sensate materials such as terpenes, vanilloids, alkyl amides, natural extracts and combinations thereof. Terpenes can include menthol and derivatives such as menthyl lactate, ethyl menthane carboxamide, and menthoyxypropanediol. Other terpenes can include camphor, eucalyptol, carvone, thymol and combinations thereof. Vanilloids can include capsaicin, zingerone, eugenol, and vanillyl butyl ether. Alkyl amides can include spilanthol, hydroxy alpha-sanschool, pellitorine and combinations thereof. Natural extracts can include peppermint oil, eucalyptol, rosemary oil, ginger oil, clove oil, capsicum, jambu extract, cinnamon oil, laricyl and combinations thereof. Additional topical sensate materials can include methyl salicylate, anethole, benzocaine, lidocane, phenol, benzyl nicotinate, nicotinic acid, cinnamic aldehyde, cinnamyl alcohol, piperine, and combinations thereof.

### 4. Humectant

The compositions of the present invention may contain a humectant. The humectants herein are selected from the group consisting of polyhydric alcohols, water soluble alkoxylated nonionic polymers, and mixtures thereof. The humectants, when used herein, are preferably used at levels of from 0.1% to 20%, more preferably from 0.5% to 5%.

Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosine phosphate, sodium lactate, pyrrolidone carbonate, glucosamine, cyclodextrin, and mixtures thereof.

Water soluble alkoxylated nonionic polymers useful herein include polyethylene glycols and polypropylene glycols having a molecular weight of up to 1000 such as those with CTFA names PEG-200, PEG-400, PEG-600, PEG-1000, and mixtures thereof.

### 5. Suspending Agent

The compositions of the present invention may further comprise a suspending agent at concentrations effective for suspending water-insoluble material in dispersed form in the compositions or for modifying the viscosity of the composition. Such concentrations range from 0.1% to 10%, preferably from 0.3% to 5.0%.

Suspending agents useful herein include anionic polymers and nonionic polymers. Useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Commercially available viscosity modifiers highly useful herein include Carbomers with tradenames Carbopol 934, Carbopol 940, Carbopol 950, Carbopol 980, and Carbopol 981, all available from B. F. Goodrich Company, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxyethyl cellulose with tradename NATROSOL, hydroxypropyl cellulose with tradename KLUCEL, cetyl hydroxyethyl cellulose with tradename POLYSURF 67, all supplied by Hercules, ethylene oxide and/or propylene oxide based polymers with tradenames CARBOWAX PEGs, POLYOX WASRs, and UCON FLUIDS, all supplied by Amerchol.

Other optional suspending agents include crystalline suspending agents which can be categorized as acyl derivatives, long chain amine oxides, and mixtures thereof. These suspending agents are described in U.S. Pat. No. 4,741,855. These preferred suspending agents include ethylene glycol esters of fatty acids preferably having from 16 to 22 carbon atoms. More preferred are the ethylene glycol stearates, both mono and distearate, but particularly the distearate containing less than 7% of the mono stearate. Other suitable suspending agents include alkanol amides of fatty acids, preferably having from 16 to 22 carbon atoms, more preferably 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin) a commercial example of which is Thixin R available from Rheox, Inc. Long chain acyl derivatives, ethylene glycol esters of long chain carboxylic acids, long chain amine oxides, and alkanol amides of long chain carboxylic acids in addition to the preferred materials listed above may be used as suspending agents.

Other long chain acyl derivatives suitable for use as suspending agents include N,N-dihydrocarbyl amido benzoic acid and soluble salts thereof (e.g., Na, K), particularly N,N-di(hydrogenated) C.sub.16, C.sub.18 and tallow amido benzoic acid species of this family, which are commercially available from Stepan Company (Northfield, III., USA).

Examples of suitable long chain amine oxides for use as suspending agents include alkyl dimethyl amine oxides, e.g., stearyl dimethyl amine oxide.
Other suitable suspending agents include primary amines having a fatty alkyl moiety having at least 16 carbon atoms, examples of which include palmitamine or stearamine, and secondary amines having two fatty alkyl moieties each having at least 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

### 6. Other Optional Components

The compositions of the present invention may contain also vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names.

The compositions of the present invention may also contain antimicrobial agents which are useful as cosmetic biocides and antidandruff agents including: water soluble components such as piroctone olamine, water insoluble components such as 3,4,4'- trichlorocarbanilide (triclocarban), triclosan and zinc pyrithione.

The compositions of the present invention may also contain chelating agents.

### H. Determining Zinc Delivery to Cells

Measurement of the free zinc levels in cells as a function of time is a direct measure of delivery of zinc across the cell wall/membrane. There are many methods available for assessing this process, many are based on use of intracellular fluorescent dyes. An exemplary method is similar to that reported by Turan *et al.* (Turan, B., Fliss, H., Désilets, M. Am. J. Physiol. 1997, 272, H2095-H2106). In this case, mamallian cells are used, although the procedure is general and can be used with all eukaryotic cells. The general methodology is as follows.

Cells are plated onto a glass cover slip. These immobilized cells are then exposed to a zinc-responsive fluorescent dye such as Fura-2 (Molecular Probes, Inc.) to load the cells with this cell-permeant fluorophore. The cells are then washed to remove dye that is not intracellular. Upon exposure to test treatments, fluorescence is measured as a function of time; in the case of Fura-2, the ratio of fluorescence intensities at 505 nm is taken in response to excitation at 340 and 380 nm. The fluorescence is conveniently measured by using an epifluorescence inverted microscope interfaced to a spectrofluorometer. Fluorescence intensity is proportional to the detected zinc level. In the case of Fura-2, it is useful after maximum fluorscent intensity is achieved to expose the cells to a chelator with a much larger affinity for zinc than calcium (e.g., N,N,N',N'-tetrakis(2-pyridylmethyl)ethylenediamine; TPEN) to assure the measured fluorescent response is due to zinc and not an artifact of calcium transport.

In the present invention, the method as described herein may be used to identify materials with zinc ionophoric behavior. Materials demonstrating this behavior increase zinc transport into cells to a larger extent than would occur with an equal level of a simple zinc salt but in the absence of the zinc ionophore. This would be considered significant (i.e., demonstrating zinc ionophoric behavior) when the increase in fluorescent intensity in the presence of the ionophore vs. exposure to a no zinc ionophoric material control is approximately 1.5-fold, preferably 2-fold higher and most preferably 2.5-fold higher.

### I. pH

Preferably, the pH of the compositions of the present invention range from 2 to 10, preferably from 3 to 9.5, more preferably from 4 to 9.

### J. Zinc Delivery to Cells Enhances Anti-Fungal Activity

Zinc pyrithione (ZPT) is an effective anti-fungal material and also has zinc ionophoric behavior. Utilizing the procedure for quantitation of intracellular zinc, it has been shown that the combination of zinc salts such as zinc sulfate or zinc oxide with ZPT dramatically increase the rate of zinc transport into model cells (human umbilical vein endothelial cells evaluated with 1 mM ZPT (0.32 ppm) and an equal weight proportion of zinc salt):

| | Rate of Zinc Transport (min⁻¹) | Antifungal activity of ZPT | |
|---|---|---|---|
| | | Amount of ZPT Required (%)* | Amount of zinc salt required (ppm) |
| ZPT Alone | 4 | 100 | 0 |
| ZnO Alone | ∼ 0 | ----- | 500 |
| ZPT + ZnO | 22 | 50 | 5 |
| ZnSO₄ Alone | ∼0 | ----- | 5000 |
| ZPT + ZnSO₄ | 18 | 50 | 5 |

| | | | |
|---|---|---|---|
| * 100% ZPT is equivalent to 8ppm (MIC of ZPT alone) | | | |

Antifungal activity is assessed microbiologically by a method as described below¹. The antifungal activity of ZPT is strongly increased by the presence of the additional zinc salts, even though these salts have very weak antifungal activity independently (i.e., high MIC values). This increase in ZPT antifungal activity is seen as a reduced level of ZPT required (50% of amount needed when tested alone) to inhibit cell growth when very low levels (5ppm) of either zinc salt is present.

Without being bound by theory, it is believed that the increased zinc transport when ZPT is combined with zinc salts is responsible for the increased antifungal activity of the combination vs. ZPT alone. This establishes the relationship between zinc transport and antifungal activity, thereby supporting the finding that zinc ionophoric materials are effective antifungal materials.

¹ The Minimum Inhibitory Concentration is indicative of anti-fungal efficacy. Generally, the lower the value of the composition, the better its anti-fungal efficacy, due to increased inherent ability of the anti-dandruff agent to inhibit the growth of microorganisms.

*Malassezia furfur* was grown in a flask containing mDixon medium (see E. Gueho, et al. Antoinie Leeuwenhoek (1996), no. 69, 337-55). Dilutions of solubilized anti-microbial active were then added to test tubes containing molten mDixon agar. *M. furfur* inoculum was added to each tube of molten agar, the tube vortexed, and the contents poured into separate sterile petri dishes. After the plates are incubated, they were observed for visible M. *furfur* growth. The lowest tested dilution of anti-microbial active that yields no growth is defined as the Minimal Inhibitory Concentration (MIC).

### Equipment/Reagents

| | |
|---|---|
| Microbe | *Malassezia furfur* (ATCC 14521) |
| Erlenmeyer flask | 250ml |
| Agar medium | 9.5ml mDixon agar per concentration per active tested |
| Solvent | water, dimethyl sulfonyl oxide ("DMSO") |
| Zinc pyridinethione | ZPT having an average particle size of 2.5µm, |
| Test tubes | 2 tubes per anti-microbial active per concentration per active tested, sterilized, size = 18mm x 150mm |
| Petri dishes | 2 dishes per anti-microbial active per concentration per active tested, sterilized, size = 15mm x 100mm |

### Experimental procedure

1) *Malassezia furfur* was grown in a 250 ml Erlenmeyer flask containing 100 ml "mDIXON" medium at 320 rpm and 30°C until turbid.
2) Selected dilutions were prepared using an appropriate dilution series, of the anti-microbial active or combination in solvent, which allowed the sample active to be solubilized prior to addition to the final test agar. For each concentration of the ZPT samples, the solvent was "DMSO"; for other samples, the solvent was water or "DMSO" or other suitable solvent.
3) 0.25 ml dilutions of anti-microbial active were added to test tubes containing 9.5 ml molten "mDIXON" agar (held at 45°C in a water bath).
4) 0.25 *M. furfur* inoculum (adjusted to 5 x 10⁵ cfu/ml by direct count) was added to each test tube of molten agar.
5) Each tube was vortexed, and the contents poured into separate petri dishes.
6) After the agar solidified, the plates inverted and incubated at 30° for 5 days.
7) The plates were then observed for visible *M. furfur* growth.

### K. Classification of Zinc-Containing Materials According to their Zinc Availability

Zinc-containing materials (ZCMs) differ with respect to how strongly the zinc ion (Zn²⁺) is held by counterions in the crystal lattice. The benefits discussed herein depend upon having available Zn²⁺. To determine which ZCMs provide sufficient labile Zn²⁺ and those that do not, a test was developed using a metallochromic dye which changes color upon coordinating Zn²⁺. The response is a binary visual assessment of whether or not the color changes indicating zinc-binding. If the color changes, the ZCM is classified as having available Zn²⁺ whereas if it does not change, the ZCM is not useful for this invention.

The method is based on the commercial metallochromic dye zincon. Zincon changes from an orange color to blue upon binding zinc and provides the basis for detecting available Zn²⁺:

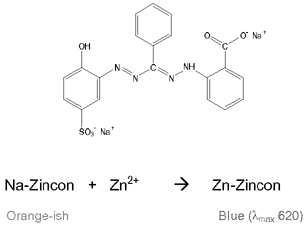

The specific procedure involves making a stock solution of zincon in ethanol (~50mg/10 ml ethanol). The ZCM is then added to water (-30 mg/10 ml water) and agitated (pH range should be 7-11). Three to four drops of zincon solution are then added to the ZCM in water, agitated and a visual assessment of color change made.

Using this methodology, the following ZCMs are examples of those that have available zinc: zinc chloride, zinc sulfate, zinc citrate, zinc oxide, zinc acetate, zinc stearate, zinc lactate, zinc salicylate, zinc arginine, zinc histadine, zinc hexaborate, zinc hydroxide, zinc oxalate, zinc monoglycerolate and the like. Examples of ZCMs not having available Zn²⁺ are zinc EDTA, zinc sulfide, zinc phytate and other materials with very tightly bound zinc.

In an embodiment of the present invention, the composition comprises from 5% to 50% of a surfactant; a zinc containing material wherein zinc availability is measured by a zinc ion reacting with a metallochromic dye zincon to give a dye color change from orange to blue. In another embodiment of the present invention, the composition comprises from 5% to 50% of a surfactant; a zinc containing material wherein zinc availability is measured by a zinc ion reacting with a metallochromic dye zincon to give a dye color change from orange to blue and a zinc ionophoric material.

### L. Methods of Manufacture

The compositions of the present invention may be prepared by any known or otherwise effective technique, suitable for providing an anti-microbial composition provided that the resulting composition provides the excellent anti-microbial benefits described herein. Methods for preparing the anti-dandruff and conditioning shampoo embodiments of the present invention include conventional formulation and mixing techniques. A method such as that described in U.S. Pat. No. 5,837,661, could be employed, wherein the anti-microbial agent of the present invention would typically be added in the same step as the silicone premix is added in the U.S. Pat. No. 5,837,661 description.

### M. Methods of Use

The compositions of the present invention may be used in direct application to the skin or in a conventional manner for cleansing skin and hair and controlling microbial infection (including fungal, viral, or bacterial infections) on the skin or scalp. The present invention may be used for treating or cleansing of the skin or hair of animals as well. Directly applied compositions, such as powders, are used by applying an effective amount of the composition, typically form 1 to 20g, to the skin; for example, to the feet. The cleansing compositions herein are useful for cleansing the hair and scalp, and other areas of the body such as underarm, feet, and groin areas and for any other area of skin in need of treatment. An effective amount of the composition, typically from 1g to 50g, preferably from 1g to 20g of the composition, for cleansing hair, skin or other area of the body, is topically applied to the hair, skin or other area that has preferably been wetted, generally with water, and then rinsed off. Application to the hair typically includes working the shampoo composition through the hair.

A preferred method for providing anti-microbial (especially anti-dandruff) efficacy with a shampoo embodiment comprises the steps of: (a) wetting the hair with water, (b) applying an effective amount of the anti-microbial shampoo composition to the hair, and (c) rinsing the anti-microbial shampoo composition from the hair using water. These steps may be repeated as many times as desired to achieve the cleansing, conditioning, and anti-microbial/anti-dandruff benefits sought.

It is also contemplated that when the anti-microbial active employed is zinc pyrithione, and/or if other optional hair growth regulating agents are employed, the anti-microbial compositions of the present invention, may, provide for the regulation of growth of the hair. The method of regularly using such shampoo compositions comprises repeating steps a, b, and c (above).

A further embodiment of the present invention comprises a method comprising the steps of (a) wetting the hair with water, (b) applying an effective amount of a shampoo composition comprising a zinc ionophore, (c) rinsing the shampoo compositions from the hair using water; (d) applying an effective amount of a conditioner composition comprising a zinc containing material according to the present invention; (e) rinsing the conditioner composition from the hair using water. In a further embodiment, this method could be conducted wherein steps d and b are reversed. In a further embodiment, steps b and d can vary and be a shampoo, hair lotions, hair sprays, hair tonics, conditioning treatments, gels, mousses and dressings, and the like. A preferred embodiment of the above mentioned method includes a shampoo composition comprising zinc pyrithione and a conditioner composition comprising zinc oxide.

A cosmetic method of improving the appearance of a scalp comprising treating the effected area with a composition comprising a zinc ionophoric material; a cosmetic method of treating fungal infections comprising treating the effected area with a composition comprising a zinc ionophoric material; a cosmetic method of treating dandruff comprising treating the effected area with a composition comprising a zinc ionophoric material; a cosmetic method of treating yeast infections comprising treating the effected area with a composition comprising a zinc ionophoric material; a cosmetic method for providing anti-dandruff efficacy comprising applying to the hair and scalp materials having zinc ionophoric behavior.

A further embodiment of the present invention comprises compositions wherein a zinc ionophoric material is in combination with a zinc containing material.

### N. Examples

The following examples further describe and demonstrate the preferred embodiments within the scope of the present invention. The examples are given solely for the purpose of illustration, and are not to be construed as limitations of the present invention since many variations thereof are possible without departing from its scope.

The composition of the invention can be made by mixing one or more selected metal ion sources and one or more metal salts of pyrithione in an appropriate media or carrier, or by adding the individual components separately to the skin or hair cleansing compositions. Useful carriers are discussed more fully above.

### 1. Topical Compositions

All exemplified compositions can be prepared by conventional formulation and mixing techniques. Component amounts are listed as weight percents and exclude minor materials such as diluents, filler, and so forth. The listed formulations, therefore, comprise the listed components and any minor materials associated with such components. As used herein, "minors" refers to those optional components such as preservatives, viscosity modifiers, pH modifiers, fragrances, foam boosters, and the like. As is apparent to one of ordinary skill in the art, the selection of these minors will vary depending on the physical and chemical characteristics of the particular ingredients selected to make the present invention as described herein. Other modifications can be undertaken by the skilled artisan without departing from the spirit and scope of this invention. These exemplified embodiments of the anti-microbial shampoo, anti-microbial conditioner, anti-microbial leave-on tonic, and anti-microbial foot powder compositions of the present invention provide excellent anti-microbial efficacy.

### Antimicrobial Shampoo - Examples 1-40

A suitable method for preparing the anti-microbial shampoo compositions described in Examples 1-40 (below) follows:
About one-third to all of the sodium laureth sulfate (added as 25wt% solution) and acid are added to a jacketed mix tank and heated to 60°C to 80°C with slow agitation to form a surfactant solution. The pH of this solution is 7.5. Sodium benzoate, Cocoamide MEA and fatty alcohols, (where applicable), are added to the tank and allowed to disperse. Ethylene glycol distearate ("EGDS") is added to the mixing vessel and allowed to melt (where applicable). After the EGDS is melted and dispersed, Kathon CG is added to the surfactant solution. The resulting mixture is cooled to 25°C to 40°C and collected in a finishing tank. As a result of this cooling step, the EGDS crystallizes to form a crystalline network in the product (where applicable). The remainder of the sodium laureth sulfate and other components, including the silicone and anti-microbial agent(s), are added to the finishing tank with agitation to ensure a homogeneous mixture. Polymers (cationic or nonionic) are dispersed in water or oils as an 0.1% to 10% dispersion and/or solution and then added to the final mix. ZnO or Zinc Hydroxy carbonate ("ZHC") can be added to a premix of surfactants or water with or without the aid of a dispersing agent via conventional powder incorporation and mixing techniques into the final mix. Adjustment of ZnO particle size can be affected by various conventional mixing techniques obvious to one skilled in the art. Once all components have been added, additional viscosity modifiers may be added, as needed, to the mixture to adjust product viscosity to the extent desired.
Examples 1-10 are not within the scope of the present invention.

| | **Weight Percent** | | | | | | | | | |
|---|---|---|---|---|---|---|---|---|---|---|
| **Components** | **Example 1** | **Example 2** | **Example 3** | **Example 4** | **Example 5** | **Example 6** | **Example 7** | **Example 8** | **Example 9** | **Example 10** |
| Ammounium Laureth Sulfate | 10.00 | 10.00 | 10.00 | 10.00 | 10.00 | 10.00 | 10.00 | 10.00 | 10.00 | 10.00 |
| Ammounium Lauryl Sulfate | 6.00 | 6.00 | 6.00 | 6.00 | 6.00 | 6.00 | 6.00 | 6.00 | 6.00 | 6.00 |
| Sodium Laureth Sulfate | | | | | | | | | | |
| Sodium Lauryl Sulfate | | | | | | | | | | |
| Cocamidopropyl Betaine | | | | | | | | | | |
| EGDS | 1.50 | 1.50 | 1.50 | 1.50 | 1.50 | 1.50 | 1.50 | 1.50 | 1.50 | 1.50 |
| CMEA | 0.800 | 0.800 | 0.800 | 0.800 | 0.800 | 0.800 | 0.800 | 0.800 | 0.800 | 0.800 |
| Cetyl Alcohol | 0.900 | 0.900 | 0.900 | 0.900 | 0.900 | 0.900 | 0.900 | 0.900 | 0.900 | 0.900 |
| Guar Hydroxy Propyltrimonium Chloride (1) | 0.500 | 0.500 | 0.500 | 0.500 | 0.500 | 0.500 | 0.500 | 0.500 | 0.500 | 0.500 |
| Guar Hydroxy Propyltrimonium Chloride (2) | | | | | | | | | | |
| Guar Hydroxy Propyltrimonium Chloride (3) | | | | | | | | | | |
| Polyquaterium-10 (4) | | | | | | | | | | |
| Polyquaterium-10 (5) | | | | | | | | | | |
| PEG-7M (6) | | | | | | | | | | |
| Dimethicone (7) | 1.35 | 1.35 | 1.35 | 1.35 | 1.35 | 1.35 | 1.35 | 1.35 | 1.35 | 1.35 |
| Dimethicone (8) | | | | | | | | | | |
| Trimethylolpropane tricaprylate/tricaprate (9) | | | | | | | | | | |
| Hydrogenated Polydecene (10) | | | | | | | | | | |
| ZPT (11) | 2.00 | 1.00 | | | | | 1.00 | 1.00 | | |
| 8-hydroxyquinoline | | | 1.00 | 2.00 | | | | 1.00 | 1.00 | |
| Zinc 8-hydroxyquinoline (12) | | | | | 1.00 | 2.00 | | | | 1.00 |
| Zinc Oxide | | | | | | | | | | |
| Zinc Carbonate Basic | | | | | | | | | | |
| Zinc Sulfate | | | | | | | 3.00 | 3.00 | 3.00 | 3.00 |
| Sodium Bicarbonate | | | | | | | | | | |
| Hydrochloric Acid | | | | | | | | | | |
| Sodium Citrate | 0.400 | 0.400 | 0.400 | 0.400 | 0.400 | 0.400 | 0.400 | 0.400 | 0.400 | 0.400 |
| Citric Acid | 0.0400 | 0.0400 | 0.0400 | 0.0400 | 0.0400 | 0.0400 | 0.0400 | 0.0400 | 0.0400 | 0.0400 |
| Magnesium Sulfate | | | | | | | | | | |
| Sodium Chloride | 0.800 | 0.800 | 0.800 | 0.800 | 0.800 | 0.800 | 0.800 | 0.800 | 0.800 | 0.800 |
| Perfume | 0.750 | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. | Q.S. |

Examples 11-20 are not within the scope of the present invention.

| | **Weight Percent** | | | | | | | | | |
|---|---|---|---|---|---|---|---|---|---|---|
| **Components** | **Example 11** | **Example 12** | **Example 13** | **Example 14** | **Example 15** | **Example 16** | **Example 17** | **Example 18** | **Example 19** | **Example 20** |
| Ammounium Laureth Sulfate | | | | | | | | | | |
| Ammounium Lauryl Sulfate | | | | | | | | | | |
| Sodium Laureth Sulfate | 10.00 | 10.00 | 10.00 | 10.00 | 10.00 | 10.00 | 10.00 | 10.00 | 10.00 | 10.00 |
| Sodium Lauryl Sulfate | 6.00 | 6.00 | 6.00 | 6.00 | 6.00 | 6.00 | 6.00 | 6.00 | 6.00 | 6.00 |
| Cocamidopropyl Betaine | | | | | | | | | | |
| EGDS | 1.50 | 1.50 | 1.50 | 1.50 | 1.50 | 1.50 | 1.50 | 1.50 | 1.50 | 1.50 |
| CMEA | 0.800 | 0.800 | 0.800 | 0.800 | 0.800 | 0.800 | 0.800 | 0.800 | 0.800 | 1.600 |
| Cetyl Alcohol | 0.600 | 0.600 | 0.600 | 0.600 | 0.600 | 0.600 | 0.600 | 0.600 | 0.600 | 0.600 |
| Guar Hydroxy Propyltrimonium Chloride (1) | 0.500 | 0.500 | 0.500 | 0.500 | 0.500 | 0.500 | 0.500 | 0.500 | 0.500 | 0.500 |
| Guar Hydroxy Propyltrimonium Chloride (2) | | | | | | | | | | |
| Guar Hydroxy Propyltrimonium Chloride (3) | | | | | | | | | | |
| Polyquaterium-10 (4) | | | | | | | | | | |
| Polyquaterium-10 (5) | | | | | | | | | | |
| PEG-7M (6) | | | | | | | | | | |
| Dimethicone (7) | 0.85 | 0.85 | 0.85 | 0.85 | 0.85 | 0.85 | 0.85 | 0.85 | 0.85 | 0.85 |
| Dimethicone (8) | | | | | | | | | | |
| Trimethylolpropane tricaprylate/tricaprate (9) | | | | | | | | | | |
| Hydrogenated Polydecene (10) | | | | | | | | | | |
| ZPT (11) | 1.00 | 1.00 | | 1.00 | 1.00 | | | 1.00 | 1.00 | 1.00 |
| 8-hydroxyquinoline | | 1.00 | 1.00 | | | 1.00 | | | | |
| Zinc 8-hydroxyquinoline (12) | | | | | | | 1.00 | | | |
| Zinc Oxide | 1.20 | 1.20 | 1.20 | 1.20 | 1.20 | 1.20 | 1.20 | 1.20 | 1.20 | 1.20 |
| Zinc Carbonate Basic | | | | | | | | | | |
| Zinc Sulfate | | | | | | | | | | |
| Sodium Bicarbonate | 0.20 | 0.20 | 0.20 | 0.10 | 0.05 | 0.05 | 0.05 | 0.25 | | 0.20 |
| Hydrochloric Acid | 0.78 | 0.78 | 0.78 | 0.53 | 0.40 | 0.40 | 0.40 | 0.91 | 0.28 | 0.78 |
| Sodium Citrate | | | | | | | | | | |
| Citric Acid | | | | | | | | | | |
| Magnesium Sulfate | 0.28 | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 | 0.800 |
| Perfume | 0.750 | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. | Q.S. |

Examples 21-24 are not within the scope of the present invention.

| | **Weight Percent** | | | | | | | | | |
|---|---|---|---|---|---|---|---|---|---|---|
| **Components** | **Example 21** | **Example 22** | **Example 23** | **Example 24** | **Example 25** | **Example 26** | **Example 27** | **Example 28** | **Example 29** | **Example 30** |
| Ammounium Laureth Sulfate | | | | | | | | | | |
| Ammounium Lauryl Sulfate | | | | | | | | | | |
| Sodium Laureth Sulfate | 10.00 | 10.00 | 10.00 | 12.50 | 10.00 | 10.00 | 10.00 | 10.00 | 10.00 | 10.00 |
| Sodium Lauryl Sulfate | 6.00 | 6.00 | 6.00 | 1.50 | 6.00 | 6.00 | 6.00 | 6.00 | 6.00 | 6.00 |
| Cocamidopropyl Betaine | | | | 2.70 | | | | 2.00 | | |
| EGDS | 1.50 | 1.50 | 1.50 | 1.50 | 1.50 | 1.50 | 1.50 | 1.50 | 1.50 | 1.50 |
| CMEA | 1.600 | 1.600 | 1.600 | 0.800 | 0.800 | 0.800 | 1.600 | 0.800 | 0.800 | 0.800 |
| Cetyl Alcohol | 0.600 | 0.600 | 0.600 | 0.600 | 0.600 | 0.600 | 0.600 | 0.600 | 0.600 | 0.600 |
| Guar Hydroxy Propyltrimonium Chloride (1) | 0.500 | | | 0.500 | 0.500 | 0.500 | 0.500 | 0.500 | 0.500 | 0.500 |
| Guar Hydroxy Propyltrimonium Chloride (2) | | 0.500 | | | | | | | | |
| Guar Hydroxy Propyltrimonium Chloride (3) | | | 0.500 | | | | | | | |
| Polyquaterium-10 (4) | | | | | | | | | | |
| Polyquaterium-10 (5) | | | | | | | | | | |
| PEG-7M (6) | 0.200 | | | | | 0.200 | 0.200 | | | |
| Dimethicone (7) | 0.85 | 0.85 | 0.85 | 0.85 | 0.85 | 0.85 | 0.85 | 0.85 | 0.85 | 0.85 |
| Dimethicone (8) | | | | | | | | | | |
| Trimethylolpropane tricaprylate/tricaprate (9) | | | | | | | | | | |
| Hydrogenated Polydecene (10) | | | | | | | | | | |
| ZPT (11) | 1.00 | 1.00 | 1.00 | 1.00 | 1.00 | 1.00 | 1.00 | 1.00 | 1.00 | 1.00 |
| 8-hydroxyquinoline | | | | | | | | | | |
| Zinc 8-hydroxyquinoline (12) | | | | | | | | | | |
| Zinc Oxide | 1.20 | 1.20 | 1.20 | 1.20 | | | | | | |
| Zinc Carbonate Basic | | | | | 1.61 | 1.61 | 1.61 | 1.61 | 0.80 | 0.40 |
| Zinc Sulfate | | | | | | | | | | |
| Sodium Bicarbonate | 0.20 | 0.20 | 0.20 | 0.20 | | | | | | |
| Hydrochloric Acid | 0.78 | 0.78 | 0.78 | 0.78 | 0.42 | 0.42 | 0.42 | 0.42 | 0.42 | 0.42 |
| Sodium Citrate | | | | | | | | | | |
| Citric Acid | | | | | | | | | | |
| Magnesium Sulfate | 0.28 | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 | 0.800 |
| Perfume | 0.750 | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. | Q.S. |

Examples 33-34 are not within the scope of the present invention.

| | **Weight Percent** | | | | | | | | | |
|---|---|---|---|---|---|---|---|---|---|---|
| **Components** | **Example 31** | **Example 32** | **Example 33** | **Example 34** | **Example 35** | **Example 36** | **Example 37** | **Example 38** | **Example 39** | **Example 40** |
| Ammounium Laureth Sulfate | | | | | | | | | | |
| Ammounium Lauryl Sulfate | | | | | | | | | | |
| Sodium Laureth Sulfate | 10.00 | 10.00 | 10.00 | 10.00 | 10.00 | 10.00 | 10.00 | 10.00 | 10.00 | 12.50 |
| Sodium Lauryl Sulfate | 6.00 | 6.00 | 6.00 | 6.00 | 6.00 | 6.00 | 6.00 | 6.00 | 6.00 | 1.50 |
| Cocamidopropyl Betaine | | | | | | | | | | 2.70 |
| EGDS | 1.50 | 1.50 | 1.50 | 1.50 | 1.50 | 1.50 | 1.50 | 1.50 | 1.50 | 1.50 |
| CMEA | 0.800 | 0.800 | 0.800 | 0.800 | 0.800 | 1.600 | 0.800 | 0.800 | 1.600 | 0.800 |
| Cetyl Alcohol | 0.600 | 0.600 | 0.600 | 0.600 | 0.600 | 0.600 | 0.600 | 0.600 | 0.600 | 0.600 |
| GuarHydroxy Propyltrimonium Chloride (1) | 0.500 | 0.500 | 0.500 | 0.500 | 0.500 | 0.500 | | | | 0.500 |
| GuarHydroxy Propyltrimonium Chloride (2) | | | | | | | | 0.500 | | |
| GuarHydroxy Propyltrimonium Chloride (3) | | | | | | | 0.500 | | 0.500 | |
| Polyquaterium-10 (4) | | | | | | | | | | |
| Polyquaterium-10 (5) | | | | | | | | | | |
| PEG-7M (6) | | | | | | | | | | |
| Dimethicone (7) | 0.85 | 0.85 | 0.85 | 0.85 | 0.85 | 0.85 | 0.85 | 0.85 | 0.85 | 0.85 |
| Dimethicone (8) | | | | | | | | | | |
| Trimethylolpropane tricaprylate/tricaprate (9) | | | | | | | | | | |
| Hydrogenated Polydecene (10) | | | | | | | | | | |
| ZPT (11) | | 0.50 | | | 2.00 | 1.00 | 1.00 | 1.00 | 1.00 | 1.00 |
| 8-hydroxyquinoline | 1.00 | | | | | | | | | |
| Zinc 8-hydroxyquinoline (12) | | | 1.00 | 2.00 | | | | | | |
| Zinc Oxide | | | | | | | | | | |
| Zinc Carbonate Basic | 1.61 | 0.80 | 1.61 | 1.61 | 1.61 | 1.61 | 1.61 | 1.61 | 1.61 | 1.61 |
| Zinc Sulfate | | | | | | | | | | |
| Sodium Bicarbonate | | | | | | | | | | |
| Hydrochloric Acid | 0.42 | 0.42 | 0.42 | 0.42 | 0.42 | 0.42 | 0.42 | 0.42 | 0.42 | 0.42 |
| Sodium Citrate | | | | | | | | | | |
| Citric Acid | | | | | | | | | | |
| Magnesium Sulfate | 0.28 | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 | 0.800 |
| Perfume | 0.750 | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 | 0.0225 |
| Water | Q.S. | Q.S. | Q.S. | Q.S. | Q.S. | Q.S. | Q.S. | Q.S. | Q.S. | Q.S. |

| | | | | | | | | | | |
|---|---|---|---|---|---|---|---|---|---|---|
| **(1)** Guar having a molecular weight of 400,000, and having a charge density of 0.84 meq/g, available from Aqualon. **(2)** Guar having a molecular weight of 400,000, and having a charge density of 2.0 meq/g, available from Aqualon. **(3)** Cationic guar Jaguar C17 available from Rhodia **(4)** Polymer JR30M available from Amerchol **(5)** Polymer LR400 available from Amerchol **(6)** Polyox WSR N-750 available from Amerchol **(7)** Viscasil 330M available from General Electric Silicones **(8)** DC1664 available from Dow Corning Silicones **(9)** Modil P43 available from Mobil. **(10)** Puresyn 6 available from Mobil. **(11)** ZPT having an average particle size of 2.5□m, available from Arch/Olin. **(12)** Zinc oxinate available from Pfaltz & Bauer | | | | | | | | | | |

### Hair Conditioning Composition - Examples 42-83

A suitable method for preparing the anti-microbial hair conditioning compositions described in Examples 42-83 (below) by conventional formulation and mixing techniques follows:
When included in the composition, polymeric materials such as polypropylene glycol are dispersed in water at room temperature to make a polymer solution, and heated up to above 70°C. Amidoamine and acid, and when present, other cationic surfactants, ester oil of low melting point oil are added in the solution with agitation. Then high melting point fatty compound, and when present, other low melting point oils and benzyl alcohol are also added in the solution with agitation. The mixture thus obtained is cooled down to below 60°C, and the remaining components such as zinc pyrithione, zinc containing material, zinc ionophoric material and silicone compound are added with agitation, and further cooled down to 30°C.

A triblender and/or mill can be used in each step, if necessary to disperse the materials. Alternatively, up to 50% of the acid can be added after cooling below 60°C.

The embodiments disclosed herein have many advantages. For example, they can provide effective anti-microbial, especially anti-dandruff, efficacy, while not deteriorating conditioning benefits such as wet hair feel, spreadability, and rinsability, as well as providing glossiness, and dry combing.
Examples 42-52 are not within the scope of the present invention.

| **Components** | **Example 42** | **Example 43** | **Example 44** | **Example 45** | **Example 46** | **Example 47** | **Example 48** | **Example 49** | **Example 50** | **Example 51** | **Example 52** |
|---|---|---|---|---|---|---|---|---|---|---|---|
| L-Glutamic Acid | 0.412 | 0.412 | 0.412 | 0.640 | 0.640 | 0.640 | 0.412 | 0.412 | 0.412 | 0.412 | 0.412 |
| Stearamidopropyldimethy lamine | 1.600 | 1.600 | 1.600 | 2.000 | 2.000 | 2.000 | 1.600 | 1.600 | 1.600 | 1.600 | 1.600 |
| Behentrimonium Chloride | | | | | | | | | | | |
| Quaterium-18 | | | | | | | | | | | |
| Cetyl Alcohol | 2.000 | 2.000 | 2.000 | 2.500 | 2.500 | 2.500 | 2.000 | 2.000 | 2.000 | 2.000 | 2.000 |
| Stearyl Alcohol | 3.600 | 3.600 | 3.600 | 4.500 | 4.500 | 4.500 | 3.600 | 3.600 | 3.600 | 3.600 | 3.600 |
| Cetearyl Alcohol | | | | | | | | | | | |
| Polysorbate 60 | | | | | | | | | | | |
| Glyceral Monostearate | | | | | | | | | | | |
| Oleyl Alcohol | | | | | | | | | | | |
| Hydroxyethylcellulose | | | | | | | | | | | |
| Peg 2M (1) | | | | | | | | | | | |
| Dimethicone (2) | | | | | | | | | | 0.200 | 0.200 |
| Dimethicone (3) | 0.500 | 0.500 | 0.500 | 0.630 | 0.630 | 0.630 | 0.500 | 0.500 | 0.500 | | |
| Cyclopentasiloxane (3) | 2.860 | 2.860 | 2.860 | 3.570 | 3.570 | 3.570 | 2.860 | 2.860 | 2.860 | | |
| Benzyl Alcohol | 0.400 | 0.400 | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 | 0.300 | 0.300 |
| Sodium Chloride | 0.010 | 0.010 | 0.010 | 0.010 | 0.010 | 0.010 | 0.010 | 0.010 | 0.010 | 0.010 | 0.010 |
| Zinc Pyrithione (4) | 2.000 | | | 1.000 | | | 0.500 | | | 2.000 | 2.000 |
| 8-Hydroxyquinoline | | 2.000 | | | 1.000 | | | 0.500 | | | |
| Zinc 8-Hydroxyquinoline (5) | | | 2.000 | | | 1.000 | | | 0.500 | | |
| Zinc Oxide | | | | | | | | | | | 1.200 |
| Zinc Carbonate Basic | | | | | | | | | | | |
| Zinc Sulfate | | | | | | | | | | | |
| Citric Acid | 0.130 | 0.130 | 0.130 | 0.130 | 0.130 | 0.130 | 0.130 | 0.130 | 0.130 | 0.130 | 0.130 |
| Kathon | | | | | | | | | | | |
| Perfume | 0.400 | 0.400 | 0.400 | 0.400 | 0.400 | 0.400 | 0.400 | 0.400 | 0.400 | 0.400 | 0.400 |
| Sodium Hydroxide | | | | | | | | | | | |
| Isopropyl Alcohol | | | | | | | | | | | |
| Water | Q.S. | Q.S. | Q.S. | Q.S. | Q.S. | Q.S. | Q.S. | Q.S. | Q.S. | Q.S. | Q.S. |

| | | | | | | | | | | | |
|---|---|---|---|---|---|---|---|---|---|---|---|
| **(1) Polyox WAR N-10 available from Amerchol Corp.** **(2) 10,000cps Dimethicone TSF451-1MA available from GE** **(3) 15/85 Dimethicone/ Cyclomethicone Blend available from GE** **(4)** ZPT having an average particle size of 2.5□m, available from Arch/Olin. **(5)** Zinc oxinate available from Pfaltz & Bauer | | | | | | | | | | | |

Examples 53-54 and 56-63 are not within the scope of the present invention.

| **Components** | **Example 53** | **Example 54** | **Example 55** | **Example 56** | **Example 57** | **Example 58** | **Example 59** | **Example 60** | **Example 61** | **Example 62** | **Example 63** |
|---|---|---|---|---|---|---|---|---|---|---|---|
| L-Glutamic Acid | 0.412 | 0.412 | 0.412 | 0.412 | 0.412 | 0.412 | 0.412 | 0.412 | 0.412 | 0.412 | 0.412 |
| Stearamidopropyldimethy lamine | 1.600 | 1.600 | 1.600 | 1.600 | 1.600 | 1.600 | 1.600 | 1.600 | 1.600 | 1.600 | 1.600 |
| Behentrimonium Chloride | | | | | | | | | | | |
| Quaterium-18 | | | | | | | | | | | |
| Cetyl Alcohol | 2.000 | 2.000 | 2.000 | 2.000 | 2.000 | 2.000 | 2.000 | 2.000 | 2.000 | 2.000 | 2.000 |
| Stearyl Alcohol | 3.600 | 3.600 | 3.600 | 3.600 | 3.600 | 3.600 | 3.600 | 3.600 | 3.600 | 3.600 | 3.600 |
| Cetearyl Alcohol | | | | | | | | | | | |
| Polysorbate 60 | | | | | | | | | | | |
| Glyceral Monostearate | | | | | | | | | | | |
| Oleyl Alcohol | | | | | | | | | | | |
| Hydroxyethylcellulose | | | | | | | | | | | |
| Peg 2M (1) | | | | | | | | | | | |
| Dimethicone (2) | 0.200 | 0.200 | 0.200 | 0.200 | 0.200 | 0.200 | 0.200 | 0.200 | 0.200 | 0.200 | 0.200 |
| Dimethicone (3) | | | | | | | | | | | |
| Cyclopentasiloxane (3) | | | | | | | | | | | |
| Benzyl Alcohol | 0.400 | 0.400 | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 | 0.300 | 0.300 |
| Sodium Chloride | 0.010 | 0.010 | 0.010 | 0.010 | 0.010 | 0.010 | 0.010 | 0.010 | 0.010 | 0.010 | 0.010 |
| Zinc Pyrithione (4) | | | 2.000 | | | 2.000 | | | | | |
| 8-Hydroxyquinoline | 2.000 | | | 2.000 | | | 2.000 | | 1.000 | 0.500 | |
| Zinc 8-Hydroxyquinoline (5) | | 2.000 | | | 2.000 | | | 2.000 | | | 1.000 |
| Zinc Oxide | 1.200 | 1.200 | | | | | | | | | |
| Zinc Carbonate Basic | | | 1.610 | 1.610 | 1.610 | | | | | | |
| Zinc Sulfate | | | | | | 3.000 | 3.000 | 3.000 | 3.000 | 3.000 | 3.000 |
| Citric Acid | 0.130 | 0.130 | 0.130 | 0.130 | 0.130 | 0.130 | 0.130 | 0.130 | 0.130 | 0.130 | 0.130 |
| Kathon | | | | | | | | | | | |
| Perfume | 0.400 | 0.400 | 0.400 | 0.400 | 0.400 | 0.400 | 0.400 | 0.400 | 0.400 | 0.400 | 0.400 |
| Sodium Hydroxide | | | | | | | | | | | |
| Isopropyl Alcohol | | | | | | | | | | | |
| Water | Q.S. | Q.S. | Q.S. | Q.S. | Q.S. | Q.S. | Q.S. | Q.S. | Q.S. | Q.S. | Q.S. |

| | | | | | | | | | | | |
|---|---|---|---|---|---|---|---|---|---|---|---|
| **(1) Polyox WAR N-10 available from Amerchol Corp.** **(2) 10,000cps Dimethicone TSF451-1MA available from GE** **(3) 15/85 Dimethicone/ Cyclomethicone Blend available from GE** **(4)** ZPT having an average particle size of 2.5□m, available from Arch/Olin. **(5)** Zinc oxinate available from Pfaltz & Bauer | | | | | | | | | | | |

Examples 64-74 are not within the scope of the present invention.

| **Components** | **Example 64** | **Example 65** | **Example 66** | **Example 67** | **Example 68** | **Example 69** | **Example 70** | **Example 71** | **Example 72** | **Example 73** | **Example 74** |
|---|---|---|---|---|---|---|---|---|---|---|---|
| L-Glutamic Acid | 0.412 | | | | | | | | | | |
| Stearamidopropyldimethy lamine | 1.600 | 1.000 | 1.000 | 1.000 | 1.000 | 1.000 | 1.000 | 1.000 | 1.000 | 1.000 | 1.000 |
| Behentrimonium Chloride | | | | | | | | | | | |
| Quaterium-18 | | 0.750 | 0.750 | 0.750 | 0.750 | 0.750 | 0.750 | 0.750 | 0.750 | 0.750 | 0.750 |
| Cetyl Alcohol | 2.000 | 0.960 | 0.960 | 0.960 | 0.960 | 0.960 | 0.960 | 0.960 | 0.960 | 0.960 | 0.960 |
| Stearyl Alcohol | 3.600 | 0.640 | 0.640 | 0.640 | 0.640 | 0.640 | 0.640 | 0.640 | 0.640 | 0.640 | 0.640 |
| Cetearyl Alcohol | | 0.500 | 0.500 | 0.500 | 0.500 | 0.500 | 0.500 | 0.500 | 0.500 | 0.500 | 0.500 |
| Polysorbate 60 | | 0.500 | 0.500 | 0.500 | 0.500 | 0.500 | 0.500 | 0.500 | 0.500 | 0.500 | 0.500 |
| Glyceral Monostearate | | 0.250 | 0.250 | 0.250 | 0.250 | 0.250 | 0.250 | 0.250 | 0.250 | 0.250 | 0.250 |
| Oleyl Alcohol | | 0.250 | 0.250 | 0.250 | 0.250 | 0.250 | 0.250 | 0.250 | 0.250 | 0.250 | 0.250 |
| Hydroxyethylcellulose | | 0.250 | 0.250 | 0.250 | 0.250 | 0.250 | 0.250 | 0.250 | 0.250 | 0.250 | 0.250 |
| Peg 2M (1) | | 0.500 | 0.500 | 0.500 | 0.500 | 0.500 | 0.500 | 0.500 | 0.500 | 0.500 | 0.500 |
| Dimethicone (2) | 0.200 | | | | | | | | 0.252 | 0.252 | 0.252 |
| Dimethicone (3) | | 0.630 | 0.630 | 0.630 | 0.630 | 0.630 | 0.630 | 0.630 | | | |
| Cyclopentasiloxane (3) | | 3.570 | 3.570 | 3.570 | 3.570 | 3.570 | 3.570 | 3.570 | | | |
| Benzyl Alcohol | 0.400 | 0.400 | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 | 0.300 | 0.300 |
| Sodium Chloride | 0.010 | | | | | | | | | | |
| Zinc Pyrithione (4) | | 2.000 | | | 1.000 | | | | 2.000 | | |
| 8-Hydroxyquinoline | | | 2.000 | | | 1.000 | | | | 2.000 | |
| Zinc 8-Hydroxyquinoline (5) | 0.500 | | | 2.000 | | | 1.000 | | | | 2.000 |
| Zinc Oxide | | | | | | | | | | | |
| Zinc Carbonate Basic | | | | | | | | | | | |
| Zinc Sulfate | 3.000 | | | | | | | | | | |
| Citric Acid | 0.130 | 0.200 | 0.200 | 0.200 | 0.200 | 0.200 | 0.200 | 0.200 | 0.200 | 0.200 | 0.200 |
| Kathon | | | | | | | | | | | |
| Perfume | 0.400 | 0.400 | 0.400 | 0.400 | 0.400 | 0.400 | 0.400 | 0.400 | 0.400 | 0.400 | 0.400 |
| Sodium Hydroxide | | | | | | | | | | | |
| Isopropyl Alcohol | | | | | | | | | | | |
| water | Q.S. | Q.S. | Q.S. | Q.S. | Q.S. | Q.S. | Q.S. | Q.S. | Q.S. | Q.S. | Q.S. |

| | | | | | | | | | | | |
|---|---|---|---|---|---|---|---|---|---|---|---|
| **(1) Polyox WAR N-10 available from Amerchol Corp.** **(2) 10,000cps Dimethicone TSF451-1MA available from GE** **(3) 15/85 Dimethicone/ Cyclomethicone Blend available from GE** **(4)** ZPT having an average particle size of 2.5□m, available from Arch/Olin. **(5)** Zinc oxinate available from Pfaltz & Bauer | | | | | | | | | | | |

Examples 76-83 are not within the scope of the present invention.

| **Components** | **Example 75** | **Example 76** | **Example 77** | **Example 78** | **Example 79** | **Example 80** | **Example 81** | **Example 82** | **Example 83** |
|---|---|---|---|---|---|---|---|---|---|
| L-Glutamic Acid | | | | | | | | | |
| Stearamidopropyldimethy lami ne | 1.000 | 1.000 | 1.000 | | | | | | |
| Behentrimonium Chloride | | | | 3.380 | 3.380 | 3.380 | 3.380 | 3.380 | 3.380 |
| Quaterium-18 | 0.750 | 0.750 | 0.750 | | | | | | |
| Cetyl Alcohol | 0.960 | 0.960 | 0.960 | 2.320 | 2.320 | 2.320 | 2.320 | 2.320 | 2.320 |
| Stearyl Alcohol | 0.640 | 0.640 | 0.640 | 4.180 | 4.180 | 4.180 | 4.180 | 4.180 | 4.180 |
| Cetearyl Alcohol | 0.500 | 0.500 | 0.500 | | | | | | |
| Polysorbate 60 | 0.500 | 0.500 | 0.500 | | | | | | |
| Glyceral Monostearate | 0.250 | 0.250 | 0.250 | | | | | | |
| Oleyl Alcohol | 0.250 | 0.250 | 0.250 | | | | | | |
| Hydroxyethylcellulose | 0.250 | 0.250 | 0.250 | | | | | | |
| Peg 2M (1) | 0.500 | 0.500 | 0.500 | | | | | | |
| Dimethicone (2) | 0.252 | 0.252 | 0.252 | | | | | | |
| Dimethicone (3) | | | | 0.630 | 0.630 | 0.630 | 0.630 | 0.630 | 0.630 |
| Cyclopentasiloxane (3) | | | | 3.570 | 3.570 | 3.570 | 3.570 | 3.570 | 3.570 |
| Benzyl Alcohol | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 |
| Sodium Chloride | | | | | | | | | |
| Zinc Pyrithione (4) | 2.000 | | | 2.000 | | | 2.000 | | |
| 8-Hydroxyquinoline | | 2.000 | | | 2.000 | | | 2.000 | |
| Zinc 8-Hydroxyquinoline (5) | | | 2.000 | | | 2.000 | | | 2.000 |
| Zinc Oxide | | | | | | | 1.200 | | |
| Zinc Carbonate Basic | 1.610 | 1.610 | 1.610 | | | | | 1.610 | |
| Zinc Sulfate | | | | | | | | | 3.000 |
| Citric Acid | 0.200 | 0.200 | 0.200 | | | | | | |
| Kathon | | | | | | | | | |
| Perfume | 0.400 | 0.400 | 0.400 | 0.300 | 0.300 | 0.300 | 0.300 | 0.300 | 0.300 |
| Sodium Hydroxide | | | | 0.014 | 0.014 | 0.014 | 0.014 | 0.014 | 0.014 |
| Isopropyl Alcohol | | | | 0.507 | 0.507 | 0.507 | 0.507 | 0.507 | 0.507 |
| Water | Q.S. | Q.S. | Q.S. | Q.S. | Q.S. | Q.S. | Q.S. | Q.S. | Q.S. |

| | | | | | | | | | |
|---|---|---|---|---|---|---|---|---|---|
| **(1) Polyox WAR N-10 available from Amerchol Corp.** **(2) 10,000cps Dimethicone TSF451-1MA available from GE** **(3) 15/85 Dimethicone/ Cyclomethicone Blend available from GE** (4) ZPT having an average particle size of 2.5□m, available from Arch/Olin. (5) Zinc oxinate available from Pfaltz & Bauer | | | | | | | | | |

### Anti-Microbial Leave-In Hair Tonic - Examples 84-90

A suitable method for preparing the anti-microbial leave-in hair tonic compositions described in Examples 84-90 (below) follows:

Add most of the formula water; with stirring, add carbomer and mix until fully dispersed. In a separate vessel, add ethanol and then molten PEG-60 hydrogenated castor oil and perfume. Transfer this to main mix tank with agitation. Add other water soluble ingredients, minors, zinc pyrithione, zinc containing materials and/or zinc ionophoric materials. Slowly add styryl silicone and let stir. Add triethanolamine slowly with stirring.
Examples 84-90 are not within the scope of the present invention.

| | **Weight Percent** | | | | | | |
|---|---|---|---|---|---|---|---|
| **Components** | **Example 84** | **Example 85** | **Example 86** | **Example 87** | **Example 88** | **Example 89** | **Example 90** |
| Carbomer | 0.50 | 0.50 | 0.50 | 0.50 | 0.50 | 0.50 | 0.50 |
| Triethanolamine | 0.30 | 0.30 | 0.30 | 0.30 | 0.30 | 0.30 | 0.30 |
| Ethanol | 25.00 | 25.00 | 25.00 | 25.00 | 25.00 | 25.00 | 25.00 |
| Zinc Pyrithione (1) | 0.10 | | | | | | |
| Zinc 8-hydroxyquinoline (2) | | 0.10 | | 0.10 | | 0.10 | |
| 8-hydroxyquinoline | | | 0.10 | | 0.10 | | 0.10 |
| Camphor | 0.05 | 0.05 | 0.05 | 0.05 | 0.05 | 0.05 | 0.05 |
| Menthol | 0.50 | 0.50 | 0.50 | 0.50 | 0.50 | 0.50 | 0.50 |
| Panthenol | 0.05 | 0.05 | 0.05 | 0.05 | 0.05 | 0.05 | 0.05 |
| Pantyl Ethyl Ether | 0.05 | 0.05 | 0.05 | 0.05 | 0.05 | 0.05 | 0.05 |
| Zinc Sulfate | | | | 0.20 | 0.20 | | |
| Zinc Oxide | | | | | | 0.20 | |
| Zinc Carbonate Basic | | | | | | | 0.20 |
| Lactic Acid | 0.05 | 0.05 | 0.05 | 0.05 | 0.05 | 0.05 | 0.05 |
| Styryl Silicone | 0.50 | 0.50 | 0.50 | 0.50 | 0.50 | 0.50 | 0.50 |
| Ceteareth- 20 | 0.15 | 0.15 | 0.15 | 0.15 | 0.15 | 0.15 | 0.15 |
| PEG-60 Hydrogenated Castor Oil | 0.15 | 0.15 | 0.15 | 0.15 | 0.15 | 0.15 | 0.15 |
| Perfume | 0.50 | 0.50 | 0.50 | 0.50 | 0.50 | 0.50 | 0.50 |
| Water | Q.S. | Q.S. | Q.S. | Q.S. | Q.S. | Q.S. | Q.S. |

| | | | | | | | |
|---|---|---|---|---|---|---|---|
| (1) ZPT having an average particle size of 2.5□m, available from Arch/Olin. (2) Zinc oxinate available from Pfaltz & Bauer | | | | | | | |

### Anti-microbial Foot Powder - Example 91-94

The foot powder composition of Examples 91-94 is prepared by thoroughly mixing the ingredients in a mixing vessel. The powder may then be ground and/or sifted if necessary. Examples 91-94 are not within the scope of the present invention.

| | **Weight Percent** | | | |
|---|---|---|---|---|
| **Components** | **Example 91** | **Example 92** | **Example 93** | **Example 94** |
| Talc | 73.25% | 73.25% | 73.25% | 73.7% |
| Calcium Propionate | 15.0 | 15.0 | 15.0 | 15.0 |
| Zinc Propionate | 5.0 | 5.0 | 5.0 | 5.0 |
| Zinc Caprylate | 5.0 | 5.0 | 5.0 | 5.0 |
| Propionic Acid | 0.25 | 0.25 | 0.25 | 0.25 |
| Zinc Sulfate | 0.50 | 0.50 | 0.50 | |
| Zinc Pyrithione (1) | 1.0 | | | |
| Zinc 8-hydroxyquinoline (2) | | 1.0 | | |
| 8-hydroxyquinoline | | | 1.0 | 1.0 |
| | 100.00 | 100.00 | 100.00 | 100.00 |

| | | | | |
|---|---|---|---|---|
| (1) ZPT having an average particle size of 2.5□m, available from Arch/Olin. (2) Zinc oxinate available from Pfaltz & Bauer | | | | |

Examples 94-98 are not within the scope of the present invention.

### Oil-in Water cream/lotion - Examples 94-98

| | **Weight Percent** | | | | |
|---|---|---|---|---|---|
| **Components** | **Example 94** | **Example 95** | **Example 96** | **Example 97** | **Example 98** |
| Oil Phase | | | | | |
| Mineral oil | 15.0 | 20.0 | 20.0 | 20.0 | 20.0 |
| Polysorbate | 2.0 | 3.0 | 3.0 | 3.0 | 3.0 |
| Aqueous Phase | | | | | |
| Zinc Oxide | 0.2 | 0.2 | 0.2 | | |
| Zinc pyrithione (1) | 1.0 | | | | |
| Zinc 8-hydroxyquinoline (2) | | 1.0 | | 1.0 | |
| 8-hydroxyquinoline | | | 1.0 | | 1.0 |
| | | | | | |
| Preservative | 0.3 | 0.3 | 0.3 | 0.3 | 0.3 |
| Perfume | 0.2 | 0.2 | 0.2 | 0.2 | 0.2 |
| Water | Q.S. | Q.S. | Q.S. | Q.S. | Q.S. |

| | | | | | |
|---|---|---|---|---|---|
| (1) ZPT having an average particle size of 2.5□m, available from Arch/Olin. (2) Zinc oxinate available from Pfaltz & Bauer | | | | | |

### Evaluation of Several Zinc lonophoric Materials - Example 99

Anti-fungal activity of materials having zinc ionophoric behavior was screened by measurement of minimum inhibitory concentrations (MIC's) against Malassezia furfur. The lower the MIC, the more potent the anti-fungal activity.

| Material | MIC (ppm) |
|---|---|
| Enterovioform | 25 |
| 5,7-D-Br-8-HQ | 25 |
| Sterosan | 100 |
| Diodoquin | 25 |
| Lasalocid | > 1000 |
| A23187 | > 13 |
| TBTDS | > 1000 |

For perspective, ZPT has an MIC of 8 ppm and several of these materials have potency that begins to approach this potency and would, therefore, be expected to provide an anti-dandruff benefit (either alone or in combination with other actives such as ZPT). There is most likely sufficient zinc in the growth medium to meet the needs of the ionophore, but additional sources of zinc may be added.

### O. Other Preferred Embodiments

Other preferred embodiments of the present invention include the following:
An embodiment of the present invention relates to a cosmetic method for delivering excess zinc to eukaryotic cells to inhibit the metabolism of the cell, the method comprising treating the cells with composition comprising a ZIM, wherein the ZIM is capable of delivering a zinc ion across a cellular membrane, wherein the zinc ionophoric material is capable of delivering a zinc ion across a cellular membrane, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof, wherein the minimum inhibitory concention (MIC) of the zinc ionophoric material is less than 500 parts per million (ppm). The ZIM is in combination with a zinc containing material as set out above such that there is an increase in the intracellular zinc level by 1.5 folds more than would occur in the absence of the ZIM. In a further embodiment, the ZIM has a potency against target microorganisms such that the minimum inhibitory concentration is below 5000 parts per million.

In a preferred embodiment, there is a sufficient quantity of zinc ions, such that they would otherwise react with a metallochromic dye zincon to give a dye color change from orange to blue.

A preferred embodiment of the present invention is a cosmetic method for treating a variety of conditions, including: athlete's foot, microbial infections, improving the appearance of a scalp, treating fungal infections, treating dandruff, treating diaper dermatitis and candidiasis, treating tinea capitis, treating yeast infections, treating onychomycosis. Such conditions are treated by applying a composition as set out hereinbefore to the affected area. In a further embodiment of the present invention, a cosmetic method of treating a condition as described above comprising treating the affected area with a composition comprising a zinc ionophoric material, wherein the ZIM is capable of delivering a zinc ion across a cellular membrane, wherein the zinc ionophoric material is capable of delivering a zinc ion across a cellular membrane, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof. A further embodiment of the present invention comprises a method of treating a condition as described above comprising treating the affected area with a composition comprising a zinc ionophoric material with a zinc containing material as set out hereinbefore.

A preferred embodiment of the present invention is a method for providing anti-dandruff efficacy comprising applying to the hair and scalp, a composition comprising a zinc ionophoric material, wherein the ZIM is capable of delivering a zinc ion across a cellular membrane, wherein the zinc ionophoric material is capable of delivering a zinc ion across a cellular membrane, wherein the zinc ionophoric material is zinc pyrithione, wherein the zinc ionophoric material is in combination with a zinc containing material, wherein the zinc containing material is a zinc-containing layered material, wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof.

In a preferred embodiment, the compositions useful in the present invention provide an anti-fungal efficacy.

## Claims

1. A cosmetic method for delivering excess zinc to eukaryotic cells to inhibit the metabolism of the cell, the method comprising treating the cells with a composition comprising a zinc ionophoric material,
wherein the zinc ionophoric material is capable of delivering a zinc ion across a cellular membrane, wherein the zinc ionophoric material is zinc pyrithione,
wherein the zinc ionophoric material is in combination with a zinc containing material,
wherein the zinc containing material is a zinc-containing layered material,
wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof,
wherein an increase in a zinc ionophoric material's antifungal activity is achieved with at least a 50% reduction in an amount of zinc ionophoric material necessary to inhibit cell growth when in the presence of 5 ppm or less of a zinc containing material;
and further wherein there is an increase in an intracellular zinc level by 1.5 fold more than would occur in the absence of the zinc ionophoric material.

2. The cosmetic method according to Claim 1 wherein the zinc ionophoric material is present as a zinc salt of the zinc ionophoric material.

3. The cosmetic method according to any of the preceding claims wherein an increase in zinc transport by 1.5 fold is demonstrated when a zinc ionophoric material is in combination with a zinc containing material to enhance antifungal activity.

4. The cosmetic method according to any of the preceding claims wherein the zinc containing material reacts with a metallochromic dye zincon to give a dye color change from orange to blue.

5. The method according to any of the preceding claims wherein the zinc-containing layered material is zinc carbonate hydroxide, hydrozincite, basic zinc carbonate and mixtures thereof, more preferably wherein the zinc-containing layered material is hydrozincite.

6. A composition comprising a zinc ionophoric material,
wherein the zinc ionophoric material is zinc pyrithione,
wherein the zinc ionophoric material is in combination with a zinc containing material,
wherein the zinc containing material is a zinc-containing layered material,
wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof,
for use in the treatment of microbial infections.

7. A composition
comprising a zinc ionophoric material,
wherein the zinc ionophoric material is zinc pyrithione,
wherein the zinc ionophoric material is in combination with a zinc containing material,
wherein the zinc containing material is a zinc-containing layered material,
wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof,
for use in the treatment of fungal infections.

8. A composition
comprising a zinc ionophoric material,
wherein the zinc ionophoric material is zinc pyrithione,
wherein the zinc ionophoric material is in combination with a zinc containing material,
wherein the zinc containing material is a zinc-containing layered material,
wherein the zinc-containing layered material is selected from the group consisting of zinc carbonate hydroxide, zinc copper carbonate, copper zinc carbonate hydroxide phyllosilicate containing zinc ions, layered double hydroxide, hydroxy double salts and mixtures thereof,
for use in the treatment of dandruff.

## Patentansprüche

1. Kosmetisches Verfahren zur Abgabe von überschüssigem Zink an eukaryotische Zellen, um den Metabolismus der Zelle zu hemmen, wobei das Verfahren das Behandeln der Zellen mit einer Zusammensetzung umfasst, die ein ionophorisches Zinkmaterial umfasst,
wobei das ionophorische Zinkmaterial in der Lage ist, ein Zinkion über eine Zellmembran abzugeben, wobei das ionophorische Zinkmaterial Zinkpyrithion ist,
wobei das ionophorische Zinkmaterial in Kombination mit einem zinkhaltigen Material vorliegt,
wobei das zinkhaltige Material ein zinkhaltiges Schichtmaterial ist,
wobei das zinkhaltige Schichtmaterial ausgewählt ist aus der Gruppe, bestehend aus Zinkcarbonathydroxid, Zinkkupfercarbonat, Kupferzinkcarbonathydroxidphyllosilicat, das Zinkionen enthält, geschichtetem Doppelhydroxid, Hydroxydoppelsalzen und Mischungen davon,
wobei eine Erhöhung der antifungalen Aktivität eines ionophorischen Zinkmaterials mit einer Reduktion von mindestens 50 % in einer Menge an ionophorischem Zinkmaterial erreicht wird, die notwendig ist, um das Zellwachstum zu hemmen, wenn 5 ppm oder weniger eines zinkhaltigen Materials vorliegen;
und wobei ferner ein Anstieg des intrazellulären Zinkspiegels um das 1,5-Fache mehr vorliegt, als in Abwesenheit des ionophorischen Zinkmaterials auftreten würde.

2. Kosmetisches Verfahren nach Anspruch 1, wobei das ionophorische Zinkmaterial als Zinksalz des ionophorischen Zinkmaterials vorliegt.

3. Kosmetisches Verfahren nach einem der vorstehenden Ansprüche, wobei eine Erhöhung des Zinktransports um das 1,5-Fache gezeigt wird, wenn ein ionophorisches Zinkmaterial in Kombination mit einem zinkhaltigen Material vorliegt, um die antifungale Aktivität zu verstärken.

4. Kosmetisches Verfahren nach einem der vorstehenden Ansprüche, wobei das zinkhaltige Material mit einem metallochromen Farbstoffzinkon reagiert, um eine Farbstofffarbänderung von orange nach blau zu ergeben.

5. Verfahren nach einem der vorstehenden Ansprüche, wobei das zinkhaltige Schichtmaterial Zinkcarbonathydroxid, Hydrozinkit, basisches Zinkcarbonat und Mischungen davon ist, wobei mehr bevorzugt das zinkhaltige Schichtmaterial Hydrozinkit ist.

6. Zusammensetzung, umfassend ein ionophorisches Zinkmaterial,
wobei das ionophorische Zinkmaterial Zinkpyrithion ist,
wobei das ionophorische Zinkmaterial in Kombination mit einem zinkhaltigen Material vorliegt,
wobei das zinkhaltige Material ein zinkhaltiges Schichtmaterial ist,
wobei das zinkhaltige Schichtmaterial ausgewählt ist aus der Gruppe, bestehend aus Zinkcarbonathydroxid, Zinkkupfercarbonat, Kupferzinkcarbonathydroxidphyllosilicat, das Zinkionen enthält, geschichtetem Doppelhydroxid, Hydroxydoppelsalzen und Mischungen davon,
zur Verwendung bei der Behandlung mikrobieller Infektionen.

7. Zusammensetzung,
umfassend ein ionophorisches Zinkmaterial,
wobei das ionophorische Zinkmaterial Zinkpyrithion ist,
wobei das ionophorische Zinkmaterial in Kombination mit einem zinkhaltigen Material vorliegt,
wobei das zinkhaltige Material ein zinkhaltiges Schichtmaterial ist,
wobei das zinkhaltige Schichtmaterial ausgewählt ist aus der Gruppe, bestehend aus Zinkcarbonathydroxid, Zinkkupfercarbonat, Kupferzinkcarbonathydroxidphyllosilicat, das Zinkionen enthält, geschichtetem Doppelhydroxid, Hydroxydoppelsalzen und Mischungen davon,
zur Verwendung bei der Behandlung von Pilzinfektionen.

8. Zusammensetzung,
umfassend ein ionophorisches Zinkmaterial,
wobei das ionophorische Zinkmaterial Zinkpyrithion ist,
wobei das ionophorische Zinkmaterial in Kombination mit einem zinkhaltigen Material vorliegt,
wobei das zinkhaltige Material ein zinkhaltiges Schichtmaterial ist,
wobei das zinkhaltige Schichtmaterial ausgewählt ist aus der Gruppe, bestehend aus Zinkcarbonathydroxid, Zinkkupfercarbonat, Kupferzinkcarbonathydroxidphyllosilicat, das Zinkionen enthält, geschichtetem Doppelhydroxid, Hydroxydoppelsalzen und Mischungen davon,
zur Verwendung bei der Behandlung von Schuppen.

## Revendications

1. Procédé cosmétique pour distribuer du zinc en excès à des cellules eucaryotes pour inhiber le métabolisme de la cellule, le procédé comprenant le traitement des cellules avec une composition comprenant un matériau ionophore de zinc,
dans lequel le matériau ionophore de zinc est susceptible de distribuer un ion zinc à travers une membrane cellulaire, dans lequel le matériau ionophore de zinc est de la pyrithione de zinc,
dans lequel le matériau ionophore de zinc est en combinaison avec un matériau contenant du zinc,
dans lequel le matériau contenant du zinc est un matériau en couches contenant du zinc,
dans lequel le matériau en couches contenant du zinc est choisi dans le groupe constitué de carbonate hydroxyde de zinc, carbonate de zinc et de cuivre, phyllosilicate de carbonate hydroxyde de cuivre zinc contenant des ions zinc, hydroxyde double en couches, sels doubles hydroxy et des mélanges de ceux-ci,
dans lequel une augmentation d'activité antifongique du matériau ionophore de zinc est obtenue avec une réduction d'au moins 50 % d'une quantité de matériau ionophore de zinc nécessaire pour inhiber une croissance cellulaire lorsqu'on est en présence de 5 ppm ou moins d'un matériau contenant du zinc ;
et dans lequel en outre il y a une augmentation d'un taux de zinc intracellulaire de 1,5 fois plus que ce qui se produirait en l'absence du matériau ionophore de zinc.

2. Procédé cosmétique selon la revendication 1 dans lequel le matériau ionophore de zinc est présent en tant que sel de zinc du matériau ionophore de zinc.

3. Procédé cosmétique selon l'une quelconque des revendications précédentes dans lequel une augmentation du transport de zinc de 1,5 fois est démontrée lorsqu'un matériau ionophore de zinc est en combinaison avec un matériau contenant du zinc pour renforcer l'activité antifongique.

4. Procédé cosmétique selon l'une quelconque des revendications précédentes dans lequel le matériau contenant du zinc réagit avec un colorant zincon métallochromique pour donner un changement de couleur de colorant de l'orange au bleu.

5. Procédé selon l'une quelconque des revendications précédentes dans lequel le matériau en couches contenant du zinc est du carbonate hydroxyde de zinc, de l'hydrozincite, du carbonate de zinc basique et des mélanges de ceux-ci, plus préférablement dans lequel le matériau en couches contenant du zinc est de l'hydrozincite.

6. Composition comprenant un matériau ionophore de zinc,
dans lequel le matériau ionophore de zinc est de la pyrithione de zinc,
dans lequel le matériau ionophore de zinc est en combinaison avec un matériau contenant du zinc,
dans lequel le matériau contenant du zinc est un matériau en couches contenant du zinc,
dans lequel le matériau en couches contenant du zinc est choisi dans le groupe constitué de carbonate hydroxyde de zinc, carbonate de zinc et de cuivre, phyllosilicate de carbonate hydroxyde de cuivre zinc contenant des ions zinc, hydroxyde double en couches, sels doubles hydroxy et des mélanges de ceux-ci,
pour utilisation dans le traitement d'infections microbiennes.

7. Composition
comprenant un matériau ionophore de zinc,
dans lequel le matériau ionophore de zinc est de la pyrithione de zinc,
dans lequel le matériau ionophore de zinc est en combinaison avec un matériau contenant du zinc,
dans lequel le matériau contenant du zinc est un matériau en couches contenant du zinc,
dans lequel le matériau en couches contenant du zinc est choisi dans le groupe constitué de carbonate hydroxyde de zinc, carbonate de zinc et de cuivre, phyllosilicate de carbonate hydroxyde de cuivre zinc contenant des ions zinc, hydroxyde double en couches, sels doubles hydroxy et des mélanges de ceux-ci,
pour utilisation dans le traitement d'infections fongiques.

8. Composition
comprenant un matériau ionophore de zinc,
dans lequel le matériau ionophore de zinc est de la pyrithione de zinc,
dans lequel le matériau ionophore de zinc est en combinaison avec un matériau contenant du zinc,
dans lequel le matériau contenant du zinc est un matériau en couches contenant du zinc,
dans lequel le matériau en couches contenant du zinc est choisi dans le groupe constitué de carbonate hydroxyde de zinc, carbonate de zinc et de cuivre, phyllosilicate de carbonate hydroxyde de cuivre zinc contenant des ions zinc, hydroxyde double en couches, sels doubles hydroxy et des mélanges de ceux-ci,
pour utilisation dans le traitement de pellicules.
